# FASCICULE DE BREVET EUROPEEN

(11) **EP 1 464 324 B1**
(45) Date de publication et mention de la délivrance du brevet: **08.11.2006**
(21) Numéro de dépôt: 04290867.3
(22) Date de dépôt: 01.04.2004
(51) Int. Cl.: A61K 8/41, A61K 8/49, A61K 8/891, A61Q 5/08, A61Q 5/10, A61Q 19/00

(54) **Composition de coloration pour matières kératiniques humaines comprenant un colorant fluorescent et un polymère conditionneur polyorganosiloxane insoluble, procédé et utilisation**
Färbemittel zum Färben von menschlicher Keratinmaterie, enthaltend einen fluoreszierenden Farbstoff und einen unlöslichen kondizionierenden Polyorganosiloxanpolymer, Verfahren und Verwendung
Dyeing composition for human keratinous material comprising a fluorescent colouring agent and an insoluble polyorganosiloxane conditioning polymer, method and use

(30) Priorité: 01.04.2003 FR 0304033
(43) Date de publication de la demande: 06.10.2004
(73) Titulaire: L'ORÉAL, 75008 Paris (FR)
(72) Inventeur: Plos, Grégory, 75011 Paris (FR); Samain, Henri, 91570 Bievres (FR)
(74) Mandataire: Wattremez, Catherine

(56) Documents cités:
- EP-A- 1 099 437
- WO-A-99/13823
- WO-A-99/13824
- WO-A-99/13846
- DE-A- 19 646 804
- US-A- 5 830 446
- US-A1- 2002 012 681

## Description

L'invention concerne une composition comprenant au moins un colorant fluorescent dans la gamme des orangés et au moins un polymère conditionneur polyorganosiloxane insoluble particulier, ainsi que les procédés et dispositif mettant en oeuvre ces compositions. Elle concerne également l'utilisation de compositions comprenant au moins un colorant fluorescent et au moins un polymère conditionneur polyorganosiloxane insoluble particulier, pour colorer avec un effet éclaircissant les matières kératiniques humaines et plus particulièrement les fibres kératiniques comme les cheveux pigmentés ou colorés artificiellement, ainsi que la peau foncée.

Il est fréquent que les personnes ayant une peau foncée désirent s'éclaircir la peau et utilisent dans ce but des compositions cosmétiques ou dermatologiques contenant des agents de blanchiment.
Les substances les plus utilisées comme agent de blanchiment sont l'hydroquinone et ses dérivés, l'acide kojique et ses dérivés, l'acide azélaïque, l'arbutine et ses dérivés, seuls ou en association avec d'autres actifs.
Toutefois ces agents ne sont pas dépourvus d'inconvénients. En particulier, il est nécessaire de les utiliser de façon prolongée et en des quantités élevées, pour obtenir un effet de blanchiment de la peau. On n'observe pas un effet immédiat à l'application de compositions les comprenant.
En outre, l'hydroquinone et ses dérivés sont utilisés en quantité efficace pour voir apparaître un effet blanchissant. En particulier, l'hydroquinone est connue pour sa cytotoxicité vis-à-vis du mélanocyte.
Par ailleurs, l'acide kojique et ses dérivés présentent l'inconvénient d'être coûteux et de ne pouvoir, pour cette raison, être utilisés en quantité importante dans des produits à large diffusion commerciale.

Il subsiste donc le besoin de compositions cosmétiques permettant d'obtenir un teint plus clair, uniforme, homogène, d'aspect naturel, ces compositions présentant une transparence satisfaisante après application sur la peau.

Dans le domaine capillaire, il existe principalement deux grands types de coloration capillaire.
Le premier est la coloration semi-permanente ou coloration directe qui fait appel à des colorants capables d'apporter à la coloration naturelle des cheveux, une modification plus ou moins marquée résistant à plusieurs shampooings. Ces colorants sont appelés colorants directs et peuvent être mis en oeuvre de deux manières différentes. Les colorations peuvent être réalisées par application directe sur les fibres kératiniques de la composition contenant le ou les colorants directs ou par application d'un mélange réalisé extemporanément d'une composition contenant le ou les colorants directs avec une composition contenant un agent décolorant oxydant qui est de préférence l'eau oxygénée. On parle alors de coloration directe éclaircissante.
Le deuxième est la coloration permanente ou coloration d'oxydation. Celle-ci est réalisée avec des précurseurs de colorants dits "d'oxydation" qui sont des composés incolores ou faiblement colorés qui une fois mélangés à des produits oxydants, au moment de l'emploi, peuvent donner naissance par un processus de condensation oxydative à des composés colorés et colorants. Il est souvent nécessaire d'associer aux bases d'oxydation et coupleurs, un ou plusieurs colorants directs afin de neutraliser ou de rabattre les nuances trop en reflets rouges, orangés ou dorés, ou au contraire d'accentuer ces reflets rouges, orangés ou dorés.
Parmi les colorants directs disponibles, les colorants directs nitrés benzéniques ne sont pas suffisamment puissants, les indoamines, les colorants quinoniques ainsi que les colorants naturels présentent une faible affinité pour les fibres kératiniques et de ce fait conduisent à des colorations qui ne sont pas assez résistantes vis à vis des différents traitements que peuvent subir les fibres, et en particulier vis à vis des shampooings.

En outre, il existe un besoin d'obtenir un effet d'éclaircissement des fibres kératiniques humaines. Cet éclaircissement est obtenu classiquement par un procédé de décoloration des mélanines du cheveu par un système oxydant, généralement constitué par du peroxyde d'hydrogène associé ou non à des persels. Ce système de décoloration présente l'inconvénient de dégrader les fibres kératiniques et d'altérer leurs propriétés cosmétiques.

La présente invention a pour objet de résoudre les problèmes mentionnés ci-dessus et notamment de proposer une composition qui présente une bonne affinité tinctoriale pour les matières kératiniques et notamment les fibres kératiniques, de bonnes propriétés de ténacité vis à vis des agents extérieurs, et en particulier vis-à-vis des shampooings, et qui permettent également d'obtenir un éclaircissement sans altération de la matière traitée, plus particulièrement la fibre kératinique.

Il a donc été trouvé de façon inattendue et surprenante que l'utilisation de colorants fluorescents ceux dans la gamme des orangés, en présence de polymères conditionneurs polyorganosiloxanes insolubles particuliers, permettait d'atteindre ces objectifs.

La présente invention a donc pour premier objet une composition comprenant, dans un milieu cosmétiquement acceptable, au moins un colorant fluorescent dans la gamme des orangés soluble dans ledit milieu et au moins un polymère conditionneur insoluble dans ledit milieu, choisi parmi les polyorganosiloxanes ne portant pas de groupement aminé ; la composition ne comprenant pas, à titre d'agent fluorescent, du 2-(2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure.

Un second objet de l'invention concerne un procédé pour colorer avec un effet éclaircissant les fibres kératiniques humaines, dans lequel on met en oeuvre les étapes suivantes :
a) on applique sur lesdites fibres une composition selon l'invention, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres.

Un autre objet de l'invention concerne l'utilisation d'une composition comprenant dans un milieu cosmétiquement acceptable, au moins un colorant fluorescent soluble dans ledit milieu, et au moins un polymère conditionneur insoluble dans ledit milieu, choisi parmi les polyorganosiloxanes ne portant pas de groupement aminé pour colorer avec un effet éclaircissant les matières kératiniques humaines.

Un dispositif à plusieurs compartiments pour la coloration et l'éclaircissement des fibres kératiniques, comprenant au moins un compartiment renfermant la composition selon l'invention, et au moins un autre compartiment renfermant une composition renfermant au moins un agent oxydant, constitue un autre objet de l'invention.

Les compositions de l'invention permettent en particulier une meilleure fixation du colorant fluorescent dans les matières kératiniques ce qui se traduit par un effet de fluorescence accru et à un effet d'éclaircissement supérieur à celui obtenu avec le colorant fluorescent utilisé seul.
On constate également une meilleure ténacité du résultat vis-à-vis des lavages ou shampooings.

Mais d'autres caractéristiques et avantages de la présente invention apparaîtront plus clairement à la lecture de la description et de l'exemple qui vont suivre.

A moins d'une indication différente, les bornes des gammes de valeurs qui sont données dans la description, sont incluses dans ces gammes.

Comme cela a été indiqué auparavant, la composition selon l'invention comprend au moins un colorant fluorescent et au moins un polymère conditionneur polyorganosiloxane insoluble particulier.

Par colorant fluorescent, on entend au sens de la présente invention un colorant qui est une molécule qui colore par elle-même, et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Un colorant fluorescent selon l'invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores, qui ne colorent pas, car il n'absorbent pas dans la lumière visible, mais uniquement dans les Ultra Violets (longueurs d'onde allant de 200 à 400 nanomètres), et transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Enfin, le colorant fluorescent mis en oeuvre dans la composition est soluble dans le milieu de la composition. Précisons que le colorant fluorescent est différent en cela d'un pigment fluorescent qui lui, n'est pas soluble dans le milieu de la composition.

Plus particulièrement, le colorant fluorescent utilisé dans le cadre de la présente invention, éventuellement neutralisé, est soluble dans le milieu de la composition à au moins 0,001 g/l, plus particulièrement au moins 0,5 g/l, de préférence au moins 1 g/l et selon un mode de réalisation encore plus préféré, au moins 5 g/l à la température comprise entre 15 et 25°C.

Par ailleurs, selon une caractéristique de l'invention, la composition ne comprend pas, à titre de colorant fluorescent, un 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure.

Conformément à un mode de réalisation encore plus particulier de l'invention, la composition ne comprend pas, à titre de colorant fluorescent, de composé choisi parmi les colorants fluorescents hétérocycliques monocationiques azoïques, azométhiniques ou méthiniques.

Les colorants fluorescents utilisés de préférence selon la présente invention sont des colorants dans la gamme des orangés.

De préférence, les colorants fluorescents de l'invention conduisent à un maximum de réflectance se situant dans la gamme de longueur d'onde allant de 500 à 650 nanomètres, et de préférence dans la gamme de longueur d'onde allant de 550 à 620 nanomètres.

Certains des colorants fluorescents selon la présente invention sont des composés connus en eux-mêmes.

A titre d'exemples de colorants fluorescents susceptibles d'être mis en oeuvre, on peut citer les colorants fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges, de préférence aux familles suivantes : les naphtalimides ; les coumarines cationiques ou non ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

Plus particulièrement, on peut citer parmi eux :
- le Jaune Brilliant B6GL commercialisé par la société SANDOZ et de structure suivante : 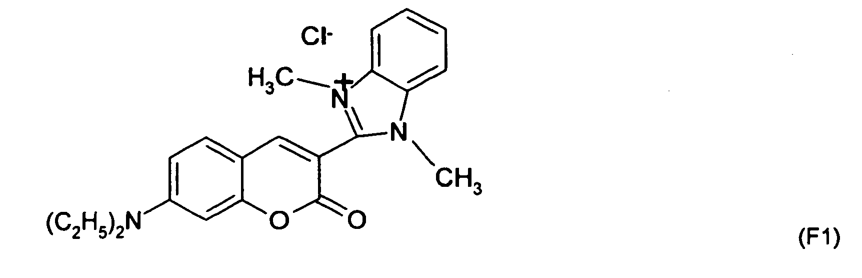
- le Basic Yellow 2, ou Auramine O commercialisé par les sociétés PROLABO, ALDRICH
ou CARLO ERBA et de structure suivante : 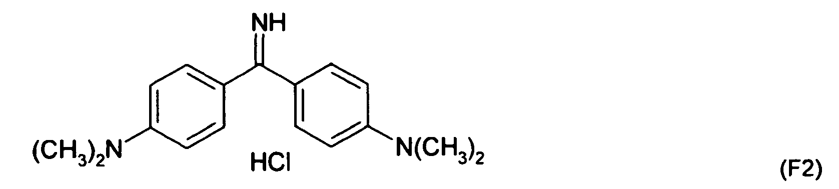 monochlorhydrate de 4,4'-(imidocarbonyl)bis(N,N-diméthylaniline) - CAS number 2465-27-2.

On peut aussi citer les composés de formule suivante : 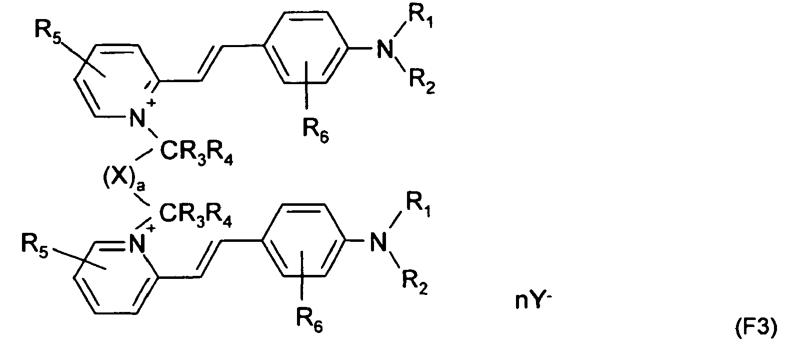 dans laquelle :
R₁, R₂, identiques ou différents, représentent :
   - un atome d'hydrogène ;
   - un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
   - un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
   - R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
   - R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
   - un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène ;
   - un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène ;
   - un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
   - un radical dicarbonyle ;
   - le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

Rappelons que les termes hétéroatomes, représentent un atome d'oxygène ou d'azote.
Parmi les groupements porteurs de tels atomes, on peut citer entre autres les groupements hydroxyle, alcoxy, carbonyle, amino, ammonium, amido (-N-CO-), carboxyle (-O-CO- ou -CO-O-).

En ce qui concerne les groupements alcényles, ces derniers comprennent une ou plusieurs liaisons carbone-carbone insaturée (-C=C-), et de préférence une seule double liaison carbone-carbone.

Dans cette formule générale, les radicaux R₁ et R₂, identiques ou non, représentent plus particulièrement :
- un atome d'hydrogène ;
- un radical alkyle comprenant 1 à 10 atomes de carbone, notamment 1 à 6 atomes de carbone, de préférence 1 à 4 atomes de carbone, éventuellement interrompu par un atome d'oxygène ou éventuellement substitué par au moins un radical hydroxyle, amino, ammonium, d'un atome de chlore ou de fluor ;
- un radical benzyle, phényle, éventuellement substitué par un radical alkyle ou alcoxy comprenant 1 à 4 atomes de carbone, de préférence 1 ou 2 atomes de carbone ;
- avec l'atome d'azote, un radical hétérocylique du type pyrrolo, pyrrolidino, imidazolino, imidazolo, imidazolium, pyrazolino, pipérazino, morpholino, morpholo, pyrazolo, triazolo, éventuellement substitué par au moins un radical alkyle linéaire ou ramifié comprenant 1 à 4 atomes de carbone éventuellement interrompu et/ou substitué par un atome d'azote et/ou d'oxygène et/ou groupement portant un atome d'azote et/ou d'oxygène.

En ce qui concerne les radicaux amino ou ammonium précités, les radicaux portés par l'atome d'azote peuvent ou non être identiques et représenter plus particulièrement un atome d'hydrogène, un radical alkyle en C₁-C₁₀, de préférence en C₁-C₄, un radical arylalkyle dans lequel, plus spécialement, le radical aryle comprend 6 atomes de carbone et le radical alkyle 1 à 10 atomes de carbone, de préférence 1 à 4 atomes de carbone.

Selon un mode de réalisation avantageux de l'invention, les radicaux R₁ et R₂, identiques ou non, représentent un atome d'hydrogène ; un radical alkyle linéaire ou ramifié en C₁-C₆ ; un radical alkyle en C₂-C₆ substitué par un radical hydroxyle ; un radical alkyle en C₂-C₆ portant un groupement amino ou ammonium ; un radical chloroalkyle en C₂-C₆ ; un radical alkyle C₂-C₆ interrompu par un atome d'oxygène ou groupement en portant un (par exemple ester) ; un radical aromatique comme les phényle, benzyle, 4-méthylphényle ; un radical hétérocyclique tel que les radicaux pyrrolo, pyrrolidino, imidazolo, imidazolino, imidazolium, pipérazino, morpholo, morphollino, pyrazolo, triazolo, éventuellement substitué par au moins un radical alkyle en C₁-C₆ ou aromatique.

De préférence, les radicaux R₁ et R₂, identiques ou différents, représentent un atome d'hydrogène, un radical alkyle linéaire ou ramifié en C₁-C₆ tels que les radicaux méthyle, éthyle, n-butyle, n-propyle ; le 2-hydroxyéthyle ; un radical alkyltriméthylammonium ou alkyltriéthylammonium, le radical alkyle étant linéaire en C₂-C₆ ; un radical (di)alkylméthylamino ou (di)alkyléthylamino, le radical alkyle étant linéaire en C₂-C₆ ; -CH₂CH₂Cl ; -(CH₂)ₙ-OCH₃ ou -(CH₂)ₙ-OCH₂CH₃ avec n nombre entier variant de 2 à 6 ; -CH₂CH₂-OCOCH₃ ; -CH₂CH₂COOCH₃.
De préférence les radicaux R₁ et R₂, identiques ou non, et de préférence identiques, représentent un radical méthyle, un radical éthyle.

Les radicaux R₁ et R₂, identiques ou différents, peuvent aussi représenter un radical hétérocyclique du type pyrrolidino, 3-amino pyrrolidino, 3-(diméthyl)amino pyrrolidino, 3-(triméthyl)amino pyrrolidino, 2,5-diméthylpyrrolo, le 1H-imidazole, 4-méthyl pipérazino, 4-benzyl pipérazino, morpholo, 3,5-(ter-butyl)-1H-pyrazolo, 1H-pyrazolo, 1H-1,2,4-triazolo.

Les radicaux R₁ et R₂, identiques ou différents, peuvent aussi représenter être reliés de manière à former un hétérocycle de formules (I) et (II) suivantes : 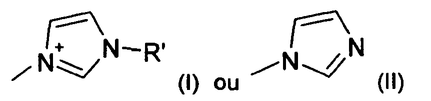 dans lesquelles R' représente un atome d'hydrogène, un radical alkyle en C₁-C₃, -CH₂CH₂OH, -CH₂CH₂OCH₃.

Conformément à un mode de réalisation plus particulier de l'invention, R₅, identiques ou non, représentent un atome d'hydrogène, un atome de fluor ou de chlore, un radical alkyle linéaire ou ramifié comprenant 1 à 4 atomes de carbone éventuellement interrompu par un atome d'oxygène ou d'azote.
Il est précisé que le substituant R₅, s'il est différent de l'hydrogène, se trouve avantageusement en position(s) 3 et/ou 5 par rapport au carbone du cycle portant l'azote substitué par les radicaux R₁ et R₂, et de préférence en position 3 par rapport à ce carbone.
Avantageusement, les radicaux R₅, identiques ou non, représentent un atome d'hydrogène ; un radical alkyle linéaire ou ramifié en C₁-C₄ ; -O-R₅₁ avec R₅₁ représentant un radical alkyle linéaire en C₁-C₄ ; -R₅₂-O-CH₃ avec R₅₂ représentant un radical alkyle linéaire en C₂-C₃ ; -R₅₃ - N (R₅₄)₂ dans laquelle R₅₃ représente un radical alkyle linéaire en C₂-C₃, R₅₄, identiques ou différents, représentent un atome d'hydrogène ou un radical méthyle.
De préférence R₅, identiques ou non, représentent l'hydrogène, un méthyle, un méthoxy, et de préférence, R₅ représente un atome d'hydrogène.

Selon un mode de réalisation particulier, les radicaux R₆, identiques ou différents, représentent un atome d'hydrogène ; un radical alkyle linéaire ou ramifié en C₁-C₄ ; -X avec X représentant un atome de chlore, de brome ou de fluor ; -R₆₁-O-R₆ avec R₆₁ représentant un radical alkyle linéaire en C₂-C₃ et R₆₂ représente le radical méthyle ; -R₆₃-N(R₆₄)₂ avec R₆₃ représentant un radical alkyle linéaire en C₂-C₃, R₆₄, identiques ou différents, représentent un atome d'hydrogène ou un radical méthyle, -N(R₆₅)₂ dans laquelle R₆₅, identiques ou différents, représentent un atome d'hydrogène, un radical alkyle linéaire en C₂-C₃ ; -NHCO R₆₆ avec R₆₆ représentant un radical alkyle en C₁-C₂, un radical chloroalkyle en C₁-C₂, un radical -R₆₇-NH₂ ou -R₆₇-NH(CH₃) ou -R₆₇-N(CH₃)₂ ou -R₆₇-N⁺(CH₃)₃ ou -R₆₇-N⁺(CH₂CH₃)₃ avec R₆₇ représentant un radical alkyle en C₁-C₂.
Il est précisé que le substituant R₆, s'il est différent de l'hydrogène, se trouve de préférence en position 2 et/ou 4 par rapport à l'atome d'azote du cycle pyridinium, et de préférence en position 4 par rapport à cet atome d'azote.

Plus particulièrement ces radicaux R₆, identiques ou non, représentent un atome d'hydrogène ou un radical méthyle ou éthyle, et de préférence, R₆ représente un atome d'hydrogène.

En ce qui concerne les radicaux R₃, R₄, ces derniers, identiques ou non, représentent avantageusement un atome d'hydrogène, un radical alkyle comprenant 1 à 4 atomes de carbone, plus spécialement un radical méthyle. De manière préférée, R₃ et R₄ représentent chacun un atome d'hydrogène.

Comme indiqué plus haut, X représente :
- un radical alkyle, linéaire ou ramifié comprenant 1 à 14 atomes de carbone, ou alcényle comprenant 2 à 14 atomes de carbone, éventuellement interrompu et/ou substitué par au moins un hétéroatome, par au moins un groupement porteur d'au moins un hétéroatome et/ou par au moins un atome d'halogène ;
- un radical hétérocyclique comprenant 5 ou 6 chaînons, éventuellement substitué par au moins un radical alkyle linéaire ou ramifié comprenant 1 à 14 atomes de carbone, par au moins un radical aminoalkyle, linéaire ou ramifié, comprenant 1 à 4 atomes de carbone, éventuellement substitué par au moins un hétéroatome ; par au moins un atome d'halogène ;
- un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement comprenant au moins un hétéroatome ;
- un radical dicarbonyle.

En outre, il est indiqué que le groupement X peut porter une ou plusieurs charges cationiques.

Ainsi, X peut représenter un radical alkyle, linéaire ou ramifié, comprenant 1 à 14 atomes de carbone, ou alcényle comprenant 2 à 14 atomes de carbone, et peut être substitué et/ou interrompu par un ou plusieurs atomes d'oxygène et/ou d'azote, et/ou par un ou plusieurs groupements porteurs d'au moins un hétéroatome , et/ou par un atome de fluor, de chlore.
Parmi les groupements de ce type, on peut citer tout particulièrement les groupements hydroxyle, alcoxy (avec notamment un radical R de type alkyle en C₁-C₄), amino, ammonium, amido, carbonyle, carboxyle (-COO-, -O-CO-) avec notamment un radical de type alkyloxy.
Notons que l'atome d'azote, s'il est présent, peut se trouver sous une forme quaternisée ou non. Dans ce cas, le ou les deux autres radicaux portés par l'atome d'azote quaternisé ou non, sont identiques ou non et peuvent être un atome d'hydrogène, un radical alkyle en C₁-C₄, de préférence le méthyle.

Selon une autre variante, le groupement X représente un radical hétérocyclique comprenant 5 ou 6 chaînons, du type imidazolo, pyrazolo, triazino, pyridino, éventuellement substitué par au moins un radical alkyle linéaire ou ramifié comprenant 1 à 14 atomes de carbone, plus particulièrement 1 à 10 atomes de carbone, de préférence de 1 à 4 atomes de carbone ; par au moins un radical aminoalkyle, linéaire ou ramifié, comprenant 1 à 10 atomes de carbone, de préférence de 1 à 4 atomes de carbone, éventuellement substitué par un groupement comprenant au moins un hétéroatome (de préférence un radical hydroxyle), ou par un atome d'halogène. A noter que le groupement amino est de préférence lié à l'hétérocycle.

Conformément à une autre possibilité, le groupement X représente un radical aromatique (comprenant de préférence 6 atomes de carbone) ou diaromatique condensé ou non (comprenant notamment de 10 à 12 atomes de carbone), séparé ou non par un radical alkyle comprenant 1 à 4 atomes de carbone, le ou les radicaux aryles étant éventuellement substitués par au moins au moins un atome d'halogène et/ou par au moins un radical alkyle comprenant 1 à 10 atomes de carbone, de préférence 1 à 4 atomes de carbone, éventuellement interrompu par au moins un atome d'oxygène et/ou d'azote, et/ou groupement comprenant au moins un hétéroatome (comme un radical carbonyle, carboxyle, amido, amino, ammonium).
Il est à noter que le radical aromatique, de préférence un radical phényle, est relié aux groupements CR₃R₄ par l'intermédiaire de liaisons en positions 1,2 ; 1,3 ou 1;4, de préférence en positions 1,3 et 1,4. Si le radical phényle relié par l'intermédiaire de liaisons en positions 1,4, porte un ou deux substituants, ce ou ces derniers sont situés de préférence en position 1,4 par rapport à l'un des groupements CR₃R₄. Si le radical phényle relié par l'intermédiaire de liaisons en positions 1,3, porte un ou deux substituants, ce ou ces derniers sont situés de préférence en position 1 et/ou 3 par rapport à l'un des groupements CR₃R₄.

Au cas où le radical est diaromatique, il est de préférence non condensé et comprend deux radicaux phényles séparés ou non par une liaison simple (soit un carbone de chacun des deux cycles) ou par un radical alkyle, de préférence de type CH₂ ou C(CH₃)₂. De manière préférée, les radicaux aromatiques ne portent pas de substituant.
Il est à noter que ledit radical diaromatique est relié aux groupements CR₃R₄ par l'intermédiaire de liaisons en positions 4,4'.

A titre d'exemples de groupements X convenables, on peut citer notamment les radicaux alkyle linéaires ou ramifiés comprenant 1 à 13 atomes de carbone tels que méthylène, éthylène, propylène, isopropylène, n-butylène, pentylène, hexylène ; le 2-hydroxypropylène, le 2-hydroxy n-butylène ; les radicaux alkylènes en C₁-C₁₃, substitués ou interrompus par un ou plusieurs atomes d'azote et/ou d'oxygène, et/ou groupements portant au moins un hétéroatome (hyroxyle, amino, ammonium, carbonyle, carboxyle, par exemple) tels que -CH₂CH₂OCH₂CH₂-, le 1,6-didéoxy-d-mannitol, -CH₂N⁺(CH₃)₂CH₂-, -CH₂CH₂N⁺(CH₃)₂-(CH₂)₆N⁺(CH₃)₂-CH₂CH₂-, CO-CO-, le 3,3-diméthylpentylène, le 2-acétoxyéthylène, le butylène1,2,3,4 tétraol ; -CH=CH- ; les radicaux aromatiques ou diaromatiques substitués par un ou plusieurs radicaux alkyle, par un ou plusieurs groupements portant au moins un hétéroatome et/ou par un ou plusieurs atomes d'halogène, tels que le 1,4-phénylène, le 1,3-phénylène, le 1,2-phénylène, le 2,6-fluorobenzène, le 4,4'-biphénylène, le 1,3-(5-méthyl benzène), le 1,2-bis(2-méthoxy)benzène, le bis(4-phényl)méthane, le 3,4 benzoate de méthyle, le 1,4-bis(amido méthyl)phényle; les radicaux de type hétérocycliques comme la pyridine, ou dérivé tel que le 2,6-bispyridine, l'imidazole, l'imidazolium, la triazine.

X représente, selon un mode de réalisation plus particulier de l'invention, un radical alkyle linéaire ou ramifié en C₁-C₁₃ ; -CH₂CH(OH)CH₂- ; -CH₂CH(Cl)CH₂- ; -CH₂CH₂-OCOCH₂- ; -CH₂CH₂COOCH₂- ; -Ra-O- Rb- avec Ra représentant un radical alkyle linéaire en C₂-C₆ et Rb représente un radical alkyle linéaire en C₁-C₂ ; - Rc-N(Rd)-Re-avec Rc représentant un radical alkyle en C₂-C₉, Rd représentant un atome d'hydrogène, un radical alkyle en C₁-C₂ et Re représentant un radical alkyle en C₁-C₆ ; -Rf-N⁺(Rg)₂-Rh- avec Rf représentant un radical alkyle linéaire en C₂-C₉, Rg, de préférence identiques, représentent un radical alkyle en C₁-C₂, Rh représente un radical alkyle linéaire en C₁-C₆ ; -CO-CO-.

X peut de plus représenter un radical imidazole, éventuellement substitué par au moins un radical alkyle comprenant 1 à 14 atomes de carbone, plus particulièrement 1 à 10 atomes de carbone, de préférence de 1 à 4, et par exemple les radicaux divalents de formule suivante : 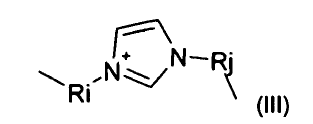 dans laquelle Ri et Rj, identiques ou non, représentent un radical alkyle linéaire en C₁C₆
X peut de même être choisi parmi les radicaux divalents dérivés de triazine suivants :

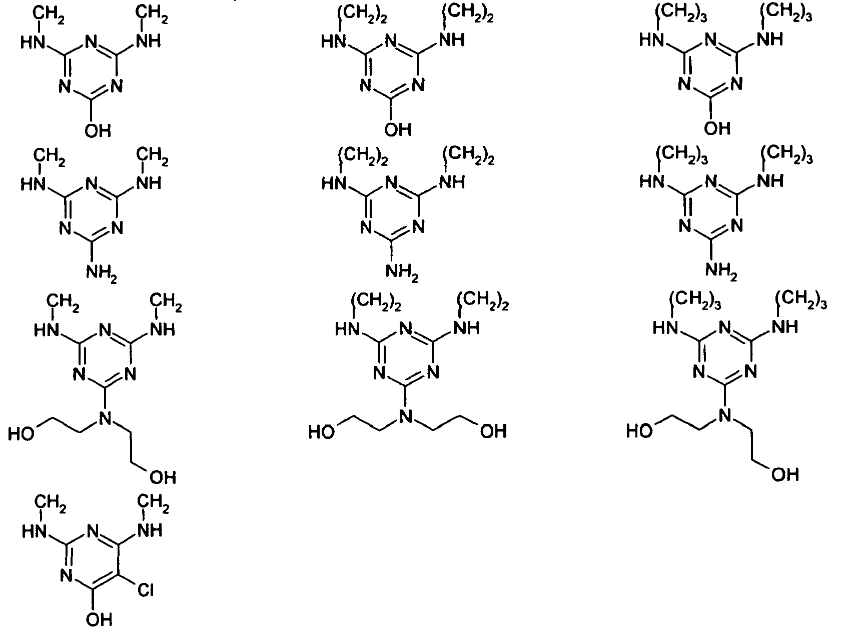

Selon une autre possibilité, X peut représenter les radicaux divalents aromatiques suivants :

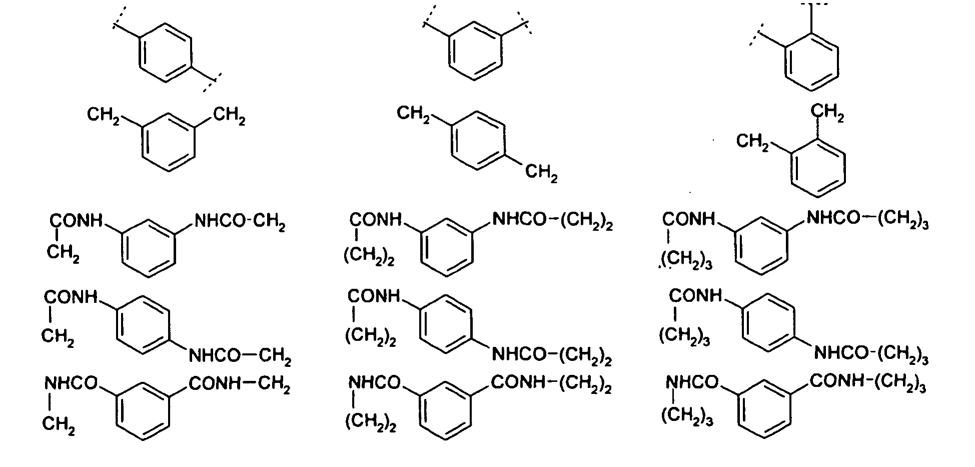

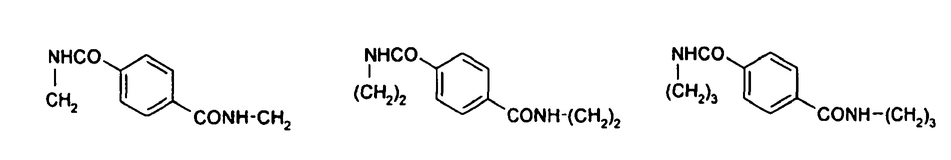

Dans la formule générale de ces composés fluorescents, Y⁻ représente un anion organique ou minéral. S'il y a plusieurs anions Y⁻, ces derniers peuvent ou non être identiques.

Parmi les anions d'origine minérale, on peut citer sans intention de s'y limiter les anions provenant d'atomes d'halogène, tels que les chlorures de préférence, les iodures, les sulfates ou bisulfates, les nitrates, les phosphates, les hydrogénophosphates, les dihydrogénophosphates, les carbonate, les bicarbonates.
Parmi les anions d'origine organique, on peut citer les anions provenant des sels d'acides mono- ou polycarboxyliques, sulfoniques, sulfuriques, saturés ou non, aromatiques ou non, éventuellement substitués par au moins un radical hydroxyle, amino, ou atomes d'halogène. A titre d'exemples non limitatifs, conviennent les acétates, hydroxyactétates, aminoacétates, (tri)chloroacétates, benzoxyactétates, propionates et dérivés portant un atome de chlore, fumarates, oxalates, acrylates, malonates, succinates, lactates, tartrates, glycollates citrates, les benzoates et dérivés portant un radical méthyle ou amino, les alkylsulfates, les tosylates, les benzènesulfonates, toluènesulfonates, etc.
De préférence, le ou les anions Y, identiques ou non, sont choisis parmi le chlore, le sulfate, le méthosulfate, l'éthosulfate.

Enfin, le nombre n, entier, est au moins égal à 2 et au plus égal au nombre de charges cationiques présentes dans le composé fluorescent.

De préférence les composés fluorescents qui viennent d'être détaillés sont des composés symétriques.

Ces composés peuvent être synthétisés en mettant en faisant réagir dans une première étape de α-picoline avec un réactif comprenant deux groupes partant qui peuvent être choisis parmi les atomes d'halogène, de préférence le brome, éventuellement le chlore,
ou les groupements de type tolylsulfonyle ou méthylesulfonyle.
Cette première étape peut avoir lieu en présence d'un solvant, bien qu'il ne soit pas obligatoire, comme par exemple le diméthylformamide.
Le nombre de moles d'α-picoline est en général voisin de 2 pour une mole de réactif comprenant les groupes partant.
En outre, la réaction est habituellement mise en oeuvre au reflux du réactif et/ou du solvant s'il est présent.
Le produit issu de cette première étape est ensuite contacté avec un aldéhyde correspondant de formule suivante : 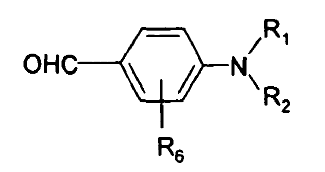 dans laquelle R₁, R₂ et R₆ ont les mêmes significations que précédemment indiquées.
Là encore, la réaction peut être effectuée en présence d'un solvant approprié, de préférence au reflux.
Il est à noter que les radicaux R₁ et R₂ de l'aldéhyde peuvent avoir la signification indiquée dans la formule générale détaillée auparavant.
Il est aussi possible de mettre en oeuvre un aldéhyde pour lequel lesdits radicaux représentent des atomes d'hydrogène et effectuer conformément à des méthodes classiques, la substitution de ces atomes d'hydrogène par des radicaux appropriés tels que décrits dans la formule générale une fois la deuxième étape terminée.

On pourra notamment se référer à des synthèses telles que décrites dans US 4256458.

Le ou les colorants fluorescents présents dans la composition selon l'invention représentent avantageusement de 0,01 à 20 % en poids, plus particulièrement de 0,05 à 10 % en poids, et de préférence de 0,1 à 5% en poids, du poids total de la composition.

Comme indiqué auparavant, la composition selon l'invention comprend, outre le colorant fluorescent, au moins un polymère conditionneur insoluble dans le milieu de la composition choisi parmi les polyorganosiloxanes ne portant pas de groupement aminé.

Il est tout d'abord rappelé qu'un agent conditionneur a notamment pour fonction l'amélioration des propriétés cosmétiques des matières kératiniques telles que les cheveux, en particulier la douceur, le démêlage, le toucher, le lissage, l'électricité statique.

En outre, par insoluble dans le milieu de la composition, on entend tout composé qui dans tout ou partie d'un domaine de concentration compris entre 0,01 et 20 % en poids, à température ambiante, dans le milieu de la composition, ne forme pas, dans ces conditions, de solution macroscopiquement isotrope transparente.

Il est à noter que conformément à un mode de réalisation particulier de l'invention, les agents conditionneurs insolubles se trouvent notamment sous une forme dispersée dans le milieu de la composition sous forme de particules ayant généralement une taille moyenne en nombre comprise entre 2 nanomètres et 100 microns, de préférence entre 30 nanomètres et 20 microns (mesurée avec un granulomètre).

Les polyorganosiloxanes (ou organosiloxanes ou silicones) non aminés insolubles présents dans la composition, peuvent se présenter en particulier sous forme d'huiles, de cires, de résines ou de gommes.
Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "Volatile Silicone 7207" par Union Carbide ou "Silbione 70045 V 2" par Rhodia Chimie, le décaméthylcyclopentasiloxane commercialisé sous le nom de "Volatile Silicone 7158" par Union Carbide, "Silbione 70045 V 5" par Rhodia Chimie, ainsi que leurs mélanges.
   On peut également citer les cyclocopolymères du type diméthylsiloxanes méthylalkylsiloxanes, tels que la "Silicone Volatile FZ 3109" commercialisée par la société Union Carbide de structure chimique : avec 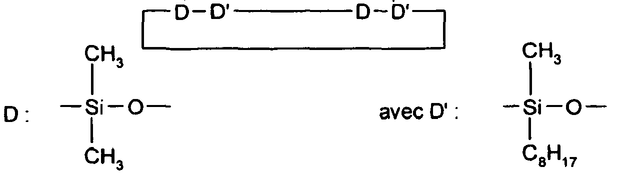 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société Toray Silicone. Des silicones entrant dans cette classe sont également décrites dans l'article publié dans Cosmetics and toiletries, Vol. 91, Jan. 76, P. 27-32 - Todd & Byers "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges. De manière avantageuse, on utilise des polyarylsiloxanes, des polyalkylarylsiloxanes, des gommes et des résines de silicones, des polyorganosiloxanes modifiés par des groupements organofonctionnels ainsi que leurs mélanges

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles Silbione^{®} des séries 47 et 70 047 ou les huiles Mirasil^{®} commercialisées par Rhodia Chimie telles que par exemple l'huile 70 047 V 500 000 ;
- les huiles de la série Mirasil commercialisées par la société Rhodia Chimie ;
- les huiles de la série 200 de la société Dow Corning telles que plus particulièrement la DC200 de viscosité 60 000 cSt (mm²/s);
- les huiles Viscasil^{®} de General Electric et certaines huiles des séries SF (SF 96, SF 18) de General Electric.

On peut également citer, et cela représente une variante très avantageuse de l'invention, les polydiméthylsiloxanes à groupements terminaux diméthylsilanol (Dimethiconol selon la dénomination CTFA) tels que les huiles de la série 48 de la société Rhodia Chimie.

Dans cette classe de polyalkylsiloxanes, on peut également citer les produits commercialisés sous les dénominations "Abil^{®} Wax 9800 et 9801" par la société Goldschmidt qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles Silbione de la série 70 641 de Rhodia Chimie ;
. les huiles des séries Rhodorsil 70 633 et 763 de Rhodia Chimie ;
. l'huile Dow Corning 556 Cosmetic Grad Fluid de Dow Corning ;
. les silicones de la série PK de Bayer comme le produit PK20 ;
. les silicones des séries PN, PH de Bayer comme les produits PN1000 et PH1000 ;
. certaines huiles des séries SF de General Electric telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants de type polydiméthylsiloxane, les gommes polydiméthylsiloxane / méthylvinylsiloxane, les polydiméthylsiloxane / diphénylsiloxane, les polydiméthylsiloxane / phénylméthylsiloxane, les polydiméthylsiloxane / diphénylsiloxane/méthylvinylsiloxane.

Des produits plus particulièrement utilisables conformément à l'invention sont des mélanges tels que :
. les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société Dow Corning ;
. les mélanges formés à partir d'une gomme polydiméthylsiloxane avec une silicone cyclique tel que le produit SF 1214 Silicone Fluid de la société General Electric, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
. les mélanges de deux PDMS de viscosités différentes, et plus particulièrement d'une gomme PDMS et d'une huile PDMS, tels que le produit SF 1236 de la société General Electric. Le produit SF 1236 est le mélange d'une gomme SE 30 définie ci-dessus ayant une viscosité de 20 m²/s et d'une huile SF 96 d'une viscosité de 5.10-⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités : R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "Dow Corning 593" ou ceux commercialisés sous les dénominations "Silicone Fluid SS 4230 et SS 4267" par la société General Electric et qui sont des silicones de structure diméthyl/triméthyl siloxane.

On peut également citer les résines du type triméthylsiloxysilicate commercialisées notamment sous les dénominations X22-4914, X21-5034 et X21-5037 par la société Shin-Etsu.

Les silicones organo-modifiées utilisables conformément à l'invention sont des silicones telles que définies précédemment et comportant dans leur structure un ou plusieurs groupements organofonctionnels fixés par l'intermédiaire d'un radical hydrocarboné. Ces composés organofonctionnels sont différents des groupements aminés.

Parmi les silicones organo-modifiées différentes de celles de formules (I) ou (II), on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société Dow Corning sous la dénomination DC 1248 et l'alkyl (C₁₂) méthicone copolyol commercialisée par la société Dow Corning sous la dénomination Q2 5200 ;
- des groupements thiols, comme les produits commercialisés sous les dénominations "GP 72 A" et "GP 71" de Genesee ;
- des groupements alcoxylés, comme le produit commercialisé sous la dénomination "Silicone Copolymer F-755" par SWS Silicones et Abil Wax 2428, 2434 et 2440 par la société Goldschmidt ;
- des groupements hydroxylés, comme les polyorganosiloxanes à fonction hydroxyalkyle décrits dans FR 8516334 ;
- des groupements acyloxyalkyle, tels que par exemple les polyorganosiloxanes décrits dans US 4957732 ;
- des groupements anioniques du type carboxylique, comme par exemple dans les produits décrits dans EP 186507 de la société Chisso Corporation, ou de type alkylcarboxyliques comme ceux présents dans le produit X-22-3701 E de la société Shin-Etsu ; 2-hydroxyalkylsulfonate ; 2-hydroxyalkylthiosulfate tels que les produits commercialisés par la société Goldschmidt sous les dénominations "Abil S201" et "Abil S255".

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans EP 412704, EP 412707, EP 640105 et WO 95/00578, EP 582152, WO 93/23009, US 4693935, US 4728571 et US 4972037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 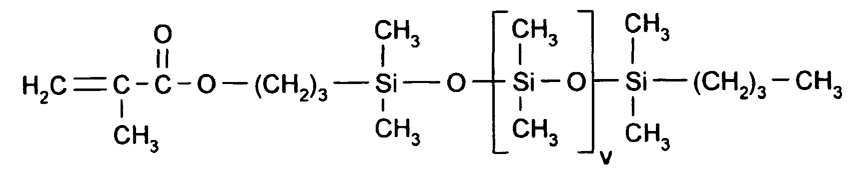 avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Il est à noter que les silicones peuvent également être utilisées sous forme d'émulsions, de nanoémulsions ou de micrémulsions.

Les silicones particulièrement préférées sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de Dow Corning en particulier celle de viscosité 60 000 cSt, des séries Silbione 70047 et 47 et plus particulièrement l'huile 70 047 V 500 000 commercialisées par la société Rhodia Chimie, les polyalkylsiloxanes à groupements terminaux diméthylsilanol tels que les diméthiconol ou les polyalkylarylsiloxanes tels que l'huile Silbione 70641 V 200 commercialisée par la société Rhodia Chimie ;
- la résine d'organopolysiloxane commercialisée sous la dénomination Dow Corning 593 ;

La teneur en polymère conditionneur insoluble est avantageusement comprise entre 0,01 à 20 % en poids, par rapport au poids de la composition, et de préférence entre 0,1 à 10 % en poids, par rapport au poids de la composition.

Le milieu cosmétiquement acceptable est généralement constitué par de l'eau ou par un mélange d'eau et d'un ou plusieurs solvants organiques usuels.

Parmi les solvants convenables, on peut citer plus particulièrement, les alcools tels que l'alcool éthylique, l'alcool isopropylique, l'alcool benzylique, et l'alcool phényléthylique, ou les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, ou encore les polyols comme le glycérol. On peut également utiliser comme solvant les polyéthylèneglycols et les polypropylèneglycols, et les mélanges de tous ces composés.

Les solvants usuels décrits ci-dessus, s'ils sont présents, représentent habituellement de 1 à 40 % en poids, plus préférentiellement de 5 à 30 % en poids, par rapport au poids total de la composition.

Le pH de la composition conforme à l'invention est généralement compris entre 3 et 12 environ, et de préférence entre 5 et 11 environ.
Il peut être ajusté à la valeur désirée au moyen d'agents acidifiants ou alcalinisants.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (A) suivante : 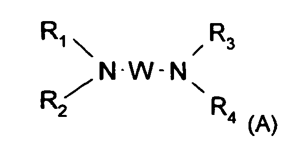 dans laquelle W est un reste propylène éventuellement substitué par un groupement hydroxyle ou un radical alkyle en C₁-C₆; R₁, R₂, R₃ et R₄, identiques ou différents, représentent un atome d'hydrogène, un radical alkyle en C₁-C₆ ou hydroxyalkyle en C₁-C₆.

Selon un mode de réalisation particulier de l'invention, la composition peut, comprendre, en plus du ou des colorants fluorescents, un ou plusieurs colorants directs non fluorescents additionnels de nature non ionique, cationique ou anionique, qui peuvent par exemple être choisis parmi les colorants benzéniques nitrés.

Conviennent notamment les colorants directs benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-((3-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène.
- le 1-(β-aminoéthyl)amino-2-nitro-4-((3-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-((β-hydroxyéthyl)amino benzène,
- la 2-nitro-4'-hydroxydiphénylamine,
- le 1-amino-2-nitro-4-hydroxy-5-méthylbenzène.

La composition conforme à l'invention peut également comprendre, en addition ou en remplacement de ces colorants benzéniques nitrés, un ou plusieurs colorants directs additionnels choisis parmi les colorants benzéniques nitrés jaunes, jaune-vert, bleus ou violets, les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

Ces colorants directs additionnels peuvent notamment être des colorants basiques parmi lesquels on peut citer plus particulièrement les colorants connus dans le COLOR INDEX, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le COLOR INDEX, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans WO 95/01772, WO 95/15144 et EP 714954 et dont le contenu fait partie intégrante de la présente invention.

Parmi les colorants directs additionnels benzéniques nitrés jaunes et jaune-vert, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxybenzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzéne,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

Parmi les colorants directs additionnels benzéniques nitrés bleus ou violets, on peut par exemple citer les composés choisis parmi :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-(β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 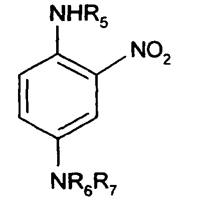
dans laquelle :
- R₆ représente un radical alkyle en C₁-C₄, un radical β-hydroxyéthyle ou β-hydroxypropyle ou γ-hydroxypropyle ;
- R₅ et R₇, identiques ou différents, représentent un radical β-hydroxyéthyle, -β-hydroxypropyle, γ-hydroxypropyle, ou β,γ-dihydroxypropyle, l'un au moins des radicaux R₆, R₇ ou R₅ représentant un radical γ-hydroxypropyle et R₆ et R₇ ne pouvant désigner simultanément un radical β-hydroxyéthyle lorsque R₆ est un radical γ-hydroxypropyle, telles que celles décrits dans FR 2692572.

Lorsqu'ils sont présents, le ou les colorants directs additionnels représentent de préférence de 0,0005 à 12 % en poids environ du poids total de la composition, et encore plus préférentiellement de 0,005 à 6 % en poids environ de ce poids.

Lorsqu'elle est destinée à la coloration d'oxydation, la composition conforme à l'invention comprend, en plus du ou des colorants fluorescents, au moins une base d'oxydation choisie parmi les bases d'oxydation classiquement utilisées pour la coloration d'oxydation et parmi lesquelles on peut notamment citer les paraphénylènediamines, les bis-phénylalkylènediamines, les para-aminophénols, les ortho-aminophénols et les bases hétérocycliques et leurs sels d'addition avec un acide ou avec un agent alcalin.

Parmi les paraphénylènediamines, on peut plus particulièrement citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylène diamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylène diamine, la N-(β-méthoxyéthyl) paraphénylènediamine et la 4'aminophényl 1-(3-hydroxy)pyrrolidine, et leurs sels d'addition avec un acide ou avec un agent alcalin.

Parmi les paraphénylènediamines citées ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide ou avec un agent alcalin.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide ou avec un agent alcalin.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide ou avec un agent alcalin.

Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide ou avec un agent alcalin.

Parmi les bases hétérocycliques, on peut plus particulièrement citer à titre d'exemple, les dérivés pyridiniques, les dérivés pyrimidiniques et les dérivés pyrazoliques et leurs sels d'addition avec un acide ou avec un agent alcalin.

Lorsqu'elles sont utilisées, la ou les bases d'oxydation représentent avantageusement de 0,0005 à 12 % en poids par rapport au poids total de la composition, et de préférence de 0,005 à 6 % en poids environ de ce poids.

Lorsqu'elle est destinée à la coloration d'oxydation, la composition conforme à l'invention peut également comprendre, en plus du ou des colorants fluorescents et des bases d'oxydation, au moins un coupleur de façon à modifier ou à enrichir en reflets les nuances obtenues en mettant en oeuvre le(s) colorant(s) fluorescent(s) et la ou les bases d'oxydation.

Les coupleurs utilisables dans la composition conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques et leurs sels d'addition avec un acide ou avec un agent alcalin.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, et leurs sels d'addition avec un acide ou avec un agent alcalin.

Lorsqu'ils sont présents, le ou les coupleurs représentent plus particulièrement de 0,0001 à 10 % en poids, et de préférence de 0,005 à 5 % en poids, par rapport au poids total de la composition.

D'une manière générale, les sels d'addition avec un acide utilisables dans le cadre des compositions de l'invention (bases d'oxydation et coupleurs) sont notamment choisis parmi les chlorhydrates, les bromhydrates, les sulfates, les citrates, les succinates, les tartrates, les tosylates, les benzènesulfonates, les lactates et les acétates.
Les sels d'addition avec un agent alcalin utilisables dans le cadre des compositions de l'invention (bases d'oxydation et coupleurs) sont notamment choisis parmi les sels d'addition avec les métaux alcalins ou alcalino-terreux, avec l'ammoniaque, avec les amines organiques dont les alcanolamines et les composés de formule (A).

La composition conforme à l'invention peut également comprendre divers adjuvants utilisés classiquement, tels que des agents tensioactifs anioniques, cationiques, non ioniques, amphotères, zwittérioniques ou leurs mélanges, des polymères anioniques, cationiques, non ioniques, amphotères, zwittérioniques autres que ceux de l'invention ou leurs mélanges, des agents épaississants minéraux, des agents antioxydants, des agents de pénétration, des agents séquestrants, des parfums, des tampons, des agents dispersants, des agents de conditionnement tels que par exemple des cations, des agents filmogènes, des céramides, des agents conservateurs, des agents stabilisants, des agents opacifiants.

Parmi les agents épaississants, on préfère plus particulièrement utiliser les systèmes épaississants à base de polymères associatifs bien connus de l'homme de l'art et notamment de nature non ionique, anionique, cationique ou amphotère.

Par ailleurs, lorsqu'ils sont présents, la teneur en tensioactifs, de préférence non ionique, anionique ou amphotère, représente 0,01 % à 30 % en poids par rapport au poids de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

La composition selon l'invention peut se présenter sous des formes diverses, telles que sous forme de liquides, de shampooings, de crèmes, de gels, ou sous toute autre forme appropriée.

Une forme particulièrement préférée selon la présente invention, la composition se trouve sous la forme d'un shampooing colorant et éclaircissant comprenant dans un milieu aqueux cosmétiquement acceptable.

Dans la composition selon l'invention, lorsqu'une ou plusieurs bases d'oxydation sont utilisées, éventuellement en présence d'un ou plusieurs coupleurs, ou lorsque le ou les colorants fluorescents sont utilisés dans le cadre d'une coloration directe éclaircissante, alors la composition conforme à l'invention peut en outre renfermer au moins un agent oxydant.
L'agent oxydant peut être choisi par exemple parmi le peroxyde d'hydrogène, le peroxyde d'urée, les bromates de métaux alcalins, les persels tels que les perborates et persulfates, et les enzymes telles que les peroxydases et les oxydo-réductases à deux ou à quatre électrons. L'utilisation du peroxyde d'hydrogène ou des enzymes est particulièrement préférée.

L'invention a également pour objet l'utilisation d'une composition comprenant, dans un milieu cosmétiquement acceptable, au moins un colorant fluorescent soluble dans ledit milieu, au moins un polymère conditionneur insoluble dans ledit milieu choisi parmi les polyorganosiloxanes ne portant pas de groupement aminé pour colorer avec un effet éclaircissant des matières kératiniques humaines.

Selon cet objet de l'invention, le colorant fluorescent peut être choisi parmi les colorants fluorescents appartenant aux familles suivantes : les naphtalimides ; les coumarines cationiques ou non ; les xanthénodiquinolizines ; les azaxanthènes ; les naphtolactames ; les azlactones ; les oxazines ; les thiazines ; les dioxazines ; les colorants fluorescents monocationiques et polycationiques de type azoïque, azométhinique, ou méthinique, seuls ou en mélanges.

A titre de composés plus particuliers, on peut citer les composés de formules F1, F2 et F3 déjà détaillées auparavant.

On peut de même utiliser les composés de structure (F4) suivante : 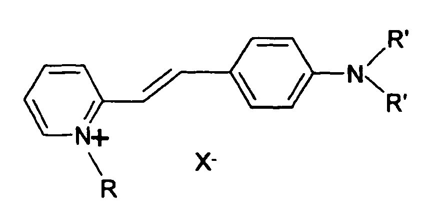 formule dans laquelle R représente un radical méthyle ou éthyle; R' représente un radical méthyle, X- un anion du type chlorure, iodure, sulfate, méthosulfate, acétate, perchlorate. A titre d'exemple de composé de ce type on peut citer le Photosensitiving Dye NK-557 commercialisé par la société UBICHEM, pour lequel R représente un radical éthyle, R' un radical méthyle et X- un iodure.

Tout ce qui a été détaillé auparavant, à propos de la nature et des teneurs des divers ingrédients mis en oeuvre dans l'invention, reste valable et ne sera pas repris dans cette partie du texte.

Selon la présente invention, on entend par matières kératiniques humaines, la peau, les cheveux, les ongles, les cils, et les sourcils, et plus particulièrement la peau foncée et les cheveux pigmentés ou colorés artificiellement.

Au sens de l'invention, on entend par peau foncée, une peau dont la luminance L* chiffrée dans le système C.I.E.L. L*a*b* est inférieure ou égale à 45 et de préférence inférieure ou égale à 40, sachant par ailleurs que L*=0 équivaut au noir et L*=100 au blanc. Les types de peau correspondant à cette luminance sont la peau africaine, la peau afro-américaine, la peau hispano-américaine, la peau indienne et la peau maghrébine.
Au sens de l'invention, on entend par cheveux pigmentés ou colorés artificiellement, des cheveux dont la hauteur de ton est inférieure ou égale à 6 (blond foncé) et de préférence inférieure ou égale à 4 (châtain).
L'éclaircissement des cheveux est évalué par la 'hauteur de ton" qui caractérise le degré
ou le niveau d'éclaircissement. La notion de "ton" repose sur la classification des nuances naturelles, un ton séparant chaque nuance de celle qui la suit ou la précède immédiatement. Cette définition et la classification des nuances naturelles sont bien connues des professionnels de la coiffure et publiée dans l'ouvrage "Sciences des traitements capillaires" de Charles ZVIAK 1988, Ed.Masson, pp.215 et 278.
Les hauteurs de ton s'échelonnent de 1 (noir) à 10 (blond clair), une unité correspondant à un ton ; plus le chiffre est élevé et plus la nuance est claire.

Un autre objet de la présente invention concerne donc un procédé de coloration avec effet éclaircissant de fibres kératiniques humaines consistant à mettre en oeuvre les étapes suivantes :
a) on applique sur les fibres kératiniques, pendant un temps suffisant pour développer la coloration et l'éclaircissement désirés, la composition selon l'invention,
b) on rince éventuellement lesdites fibres,
c) éventuellement on lave au shampooing et on rince lesdites fibres,
d) on sèche ou on laisse sécher les fibres.

La présente invention a en outre pour objet un procédé pour colorer avec un effet éclaircissant une peau foncée dans lequel on applique sur la peau, la composition qui vient d'être décrite, puis à sécher ou laisser sécher la peau. De préférence, cette composition ne comprend ni base d'oxydation ni coupleur et n'est pas mise en oeuvre en présence d'un agent oxydant.

Tout ce qui a été précédemment décrit concernant les divers éléments constitutifs de la composition reste valable et l'on pourra s'y reporter.

Notamment, les procédés selon l'invention sont appropriés pour traiter des fibres kératiniques humaines, et notamment les cheveux, pigmentées ou colorées artificiellement, ou encore de la peau foncée.
Plus particulièrement, les fibres qui peuvent être avantageusement traitées par le procédé selon l'invention, présentent une hauteur de ton est inférieure ou égale à 6 (blond foncé) et de préférence inférieure ou égale à 4 (châtain).
De plus, une peau foncée susceptible d'être traitée conformément à l'invention, présente une luminance L*, chiffrée dans le système C.I.E.L. L*a*b*, inférieure ou égale à 45 et de préférence inférieure ou égale à 40.

Selon un premier mode de réalisation de l'invention, le procédé de coloration avec effet éclaircissant des fibres est mis en oeuvre avec une composition ne comprenant pas de colorants d'oxydation ni de coupleur et en l'absence d'agent oxydant.

Selon un deuxième mode de réalisation de l'invention, le procédé de coloration avec effet éclaircissant des fibres est mis en oeuvre avec une composition ne comprenant pas de colorants d'oxydation ni de coupleur, mais en présence d'agent(s) oxydant(s).

Selon une première variante de ces procédés de coloration conformes à l'invention, on applique sur les fibres, et notamment les cheveux, au moins une composition telle que définie précédemment, pendant un temps suffisant pour développer la coloration et l'éclaircissement désirés, après quoi on rince, on lave éventuellement au shampooing, on rince à nouveau et on sèche.

Selon une deuxième variante de ces procédés de teinture conformes à l'invention, on applique sur les fibres, et notamment les cheveux au moins une composition telle que définie précédemment sans rinçage final.

Selon une troisième variante de procédé de coloration conforme à l'invention, le procédé de teinture comporte une étape préliminaire consistant à stocker sous forme séparée, d'une part, une composition selon l'invention comprenant éventuellement au moins une base d'oxydation et/ou un coupleur et, d'autre part, une composition comprenant, dans un milieu cosmétiquement acceptable, au moins un agent oxydant, puis à procéder à leur mélange au moment de l'emploi avant d'appliquer ce mélange sur les fibres kératiniques, et notamment les cheveux, pendant un temps suffisant pour développer la coloration désirée, après quoi on rince les fibres, on les lave éventuellement au shampooing, on les rince à nouveau et on les sèche.

Le temps nécessaire au développement de la coloration et à l'obtention de l'effet éclaircissant sur les fibres, notamment les cheveux, est d'environ 5 à 60 minutes et plus particulièrement d'environ 5 à 40 minutes.

La température nécessaire au développement de la coloration et à l'obtention de l'effet éclaircissant est généralement comprise entre la température ambiante (15 à 25°C) et 80°C et plus particulièrement entre 15 et 40°C.

Un autre objet de l'invention est un dispositif à plusieurs compartiments pour la coloration avec effet éclaircissant des fibres kératiniques et notamment des cheveux, comprenant au moins un compartiment renfermant une composition selon l'invention, et au moins un autre compartiment renfermant une composition comprenant au moins un agent oxydant. Ce dispositif peut être équipé d'un moyen permettant de délivrer sur les fibres le mélange souhaité, tel que les dispositifs décrits dans le brevet FR 2586913.

Il est à noter que la composition selon l'invention, si elle est utilisée pour traiter des fibres kératiniques, telles que des cheveux châtains par exemple, permet d'atteindre les résultats suivants :
Si l'on mesure la réflectance des cheveux lorsqu'on les irradie avec de la lumière visible dans la gamme de longueurs d'onde allant de 400 à 700 nanomètres, et que l'on compare les courbes de réflectance en fonction de la longueur d'onde, des cheveux traités avec la composition de l'invention et des cheveux non traités, on constate que la courbe de réflectance correspondant aux cheveux traités, dans une gamme de longueur d'onde allant de 500 à 700 nanomètres, est supérieure à celle correspondant aux cheveux non traités.
Cela signifie que, dans la gamme de longueur d'onde allant de 500 à 700 nanomètres, et de préférence de 540 à 700 nanomètres, il existe au moins une plage où la courbe de réflectance correspondant aux cheveux traités est supérieure à la courbe de réflectance correspondant aux cheveux non traités. On entend par "supérieure", un écart d'au moins 0,05% de réflectance, et de préférence d'au moins 0,1%.
Il est précisé toutefois qu'il peut exister dans la gamme de longueur d'onde allant de 500 à 700 nanomètres, et de préférence de 540 à 700 nanomètres, une ou plusieurs plages
où la courbe de réflectance correspondant aux fibres traitées est soit superposable, soit inférieure à la courbe de réflectance correspondant aux fibres non traitées.

De préférence, la longueur d'onde où l'écart est maximal entre la courbe de réflectance des cheveux traités et celle des cheveux non traités, se situe dans la gamme de longueur d'onde allant de 500 à 650 nanomètres, et de préférence dans la gamme de longueur d'onde allant de 550 à 620 nanomètres.

En outre, et de préférence, la composition selon l'invention est susceptible d'éclaircir les cheveux et la peau dans une nuance qui, chiffrée dans le système C.I.E.L L*a*b* présente une variable b* supérieure ou égale à 6, avec un rapport b*/valeur absolue de a*, supérieur à 1,2 selon le test de sélection décrit ci-dessous.

### Test de sélection

La composition est appliquée sur des fibres kératiniques châtain, plus particulièrement des cheveux, à raison de 10 grammes de composition pour 1 gramme de fibres châtain. La composition est étalée de façon à recouvrir l'ensemble des fibres. On laisse la composition agir pendant 20 minutes à température ambiante (20 à 25°C). Les fibres sont ensuite rincées à l'eau puis lavées avec un shampooing à base de lauryléther sulfate. Elles sont ensuite séchées. On mesure alors les caractéristiques spectrocolorimétriques des fibres pour en déterminer les coordonnées L*a*b*.
Dans le système C.I.E.L L*a*b*, a* et b* indiquent deux axes de couleurs, a* indique l'axe de couleur vert/rouge (+a* est rouge, -a* est vert) et b* l'axe de couleur bleu/jaune (+b* est jaune et -b* est bleu) ; des valeurs proches de zéro pour a* et b* correspondent à des nuances grises.

L'exemple qui suit est destiné à illustrer l'invention sans pour autant en limiter la portée.

### EXEMPLE

### Composé fluorescent

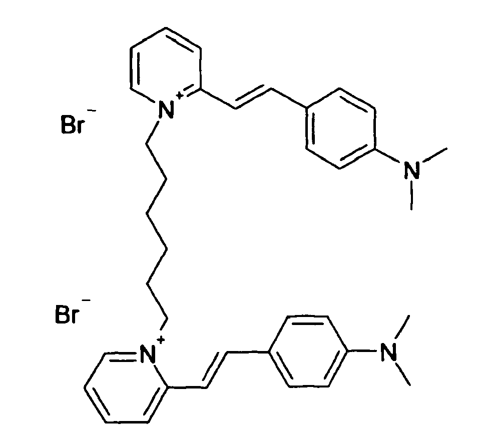

On fait réagir 93 g de 2-picoline avec 120g de 1,6 dibromohexane dans le diméthylformamide à 110°C pendant 5 heures.
On récupère le produit précipité, et on le filtre.
On solubilise 109 g du produit obtenu précédemment dans du méthanol et l'on ajoute 82,82 g de p-diméthylaminobenzaldéhyde en deux fois, en présence de pyrrolidine.
On laisse ensuite pendant 30 minutes.
On récupère le produit sous forme précipitée.

Analyse par spectroscopie de masse : 266.
Analyse élémentaire : C : 62,43 % ; H : 6,40 % ; Br : 23,07 % ; N : 8,09 %.
La formule est la suivante C₃₆H₄₄N₄.2Br.

### Composition

On prépare la composition suivante (les pourcentages sont exprimés en poids de matière active) :

| | |
|---|---|
| Composé fluorescent | 0,6 % |
| Mirasil 70047 V 500000 (Rhodia Chimie) | 0,25 % |
| Lauryléther sulfate de sodium (2,2 OE) | 10 % |
| Eau distillée | Qsp 100 % |

La composition est appliquée sur une mèche de cheveux châtains naturels de hauteur de ton 4, avec un temps de pose de 20minutes, un rinçage final et un séchage au casque pendant 30 minutes.

On obtient une mèche de cheveux avec un net effet d'éclaircissement.

## Revendications

1. Composition, **caractérisée en ce qu**'elle comprend, dans un milieu cosmétiquement acceptable, au moins un colorant fluorescent dans la gamme des orangés, soluble dans ledit milieu, au moins un polymère conditionneur insoluble dans ledit milieu, choisi parmi les polyorganosiloxanes ne portant pas de groupement aminé ; la composition ne comprenant pas, à titre d'agent fluorescent, du 2-[2-(4-dialkylamino)phényl éthényl]-1 alkyl pyridinium dans laquelle le radical alkyle du noyau pyridinium représente un radical méthyle, éthyle, celui du noyau benzénique représente un radical méthyle et dans laquelle le contre ion est un halogénure.

2. Composition selon l'une quelconque des revendications précédentes, **caractérisée en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

3. Composition selon l'une quelconque des revendications précédentes, **caractérisée en ce que** le colorant fluorescent est choisi parmi colorants fluorescents appartenant aux familles suivantes : les naphtalimides ; les coumarines cationiques ou non ; les xanthénodiquinolizines ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

4. Composition l'une quelconque des revendications précédentes, **caractérisée en ce que** le composé fluorescent est de formule suivante : 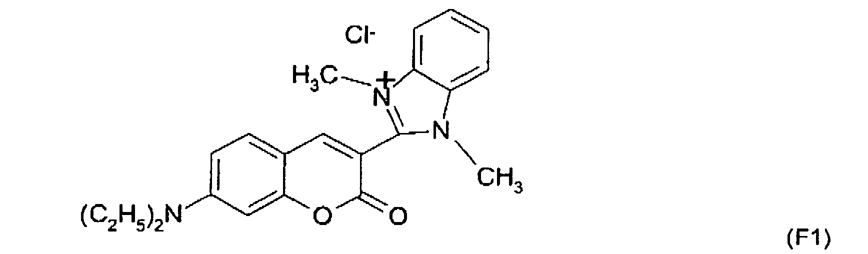 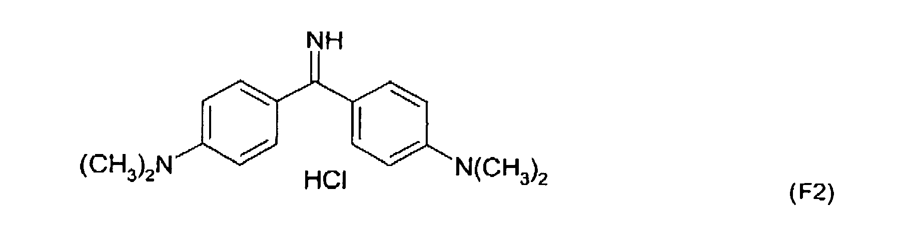 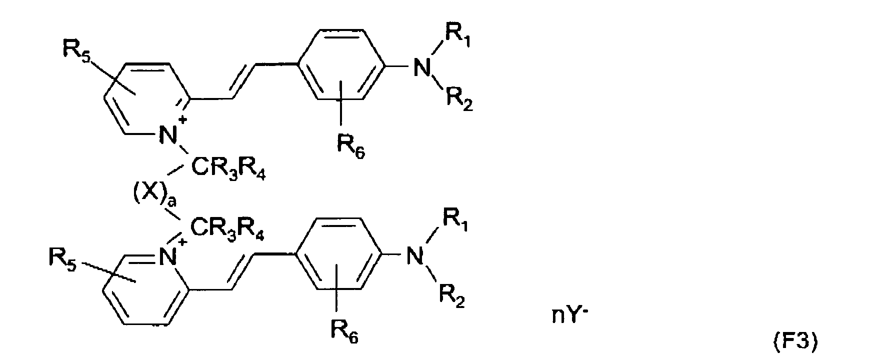 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène ;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogène;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène;
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent.

5. Composition selon l'une quelconque des revendications précédentes, **caractérisée en ce que** le ou les colorants fluorescents sont présents dans une concentration pondérale comprise entre 0,01 et 20% en poids, plus particulièrement comprise entre 0,05 à 10% en poids, de préférence comprise entre 0,1 à 5% en poids, par rapport au poids total de la composition.

6. Composition selon l'une quelconque des revendications précédentes, **caractérisée en ce que** le polymère conditionneur insoluble est choisi parmi les silicones sous forme d'huiles, de cires, de résines ou de gommes, comme les silicones volatiles cycliques comportant de 3 à 7 atomes de silicium, les cyclocopolymères, les silicones volatiles linéaires ayant 2 à 9 atomes de silicium, les silicones non volatiles du type des polyalkylsiloxanes, polyarylsiloxanes, polyalkylarylsiloxanes, polyorgano-siloxanes modifiés par des groupements organofonctionnels comme des groupements polyéthylèneoxy et/ou polypropylèneoxy comportant éventuellement des groupements alkyle, des groupements thiols, des groupements alcoxylés, des groupements hydroxylés, des groupements acyloxyalkyles, des groupements anioniques du type carboxyliques, sulfonates, thiosulfates ; des silicones greffées comprenant une partie polysiloxane et une partie constituée d'une chaîne organique non-siliconée, les résines polydiorganosiloxanes, d'organopolysiloxanes, triméthylsiloxysilicate, seuls ou en mélange.

7. Composition selon l'une des revendications précédentes, **caractérisée en ce que** la teneur en polymère conditionneur insoluble représente 0,01 à 20 % en poids par rapport au poids de la composition, plus particulièrement 0,1 à 10 % en poids par rapport à la même référence.

8. Composition selon l'une des revendications précédentes, **caractérisée en ce qu**'elle comprend au moins un tensioactif non ionique, anionique ou amphotère.

9. Composition selon la revendication précédente, **caractérisée en ce que** la teneur en tensioactif représente 0,01 à 30 % en poids par rapport au poids total de la composition.

10. Composition selon l'une quelconque des revendications précédentes, **caractérisée en ce qu**'elle comprend en outre au moins un colorant direct non fluorescent additionnel de nature non ionique, cationique ou anionique.

11. Composition selon la revendication 10, **caractérisée par le fait que** les colorants directs additionnels sont choisis parmi les colorants benzéniques nitrés, les colorants azoïques, anthraquinoniques, naphtoquinoniques, benzoquinoniques, phénotiaziniques, indigoïdes, xanthéniques, phénanthridiniques, phtalocyanines, ainsi que les colorants dérivés du triarylméthane, ou leurs mélanges.

12. Composition selon l'une des revendications 10 ou 11, **caractérisée en ce que** le ou les colorants directs additionnels représentent de 0,0005 à 12 % en poids, de préférence de 0,005 à 6 % en poids, du poids total de la composition.

13. Composition selon l'une quelconque des revendications précédentes, **caractérisée en ce qu'**elle se présente sous la forme d'un shampooing éclaircissant et colorant

14. Composition selon l'une quelconque des revendications précédentes, **caractérisée en ce qu**'elle comprend au moins une base d'oxydation choisie parmi les paraphénylènediamines, les bis-phénylalkylènediamines, les para-aminophénols, les ortho-aminophénols et les bases hétérocycliques ou leurs sels d'addition avec un acide ou avec un agent alcalin.

15. Composition selon la revendication précédente, **caractérisée en ce que** la ou les bases d'oxydation représentent 0,0005 à 12 % en poids, plus particulièrement 0,005 à 6 % en poids, du poids total de la composition.

16. Composition selon l'une quelconque des revendications 14 ou 15, **caractérisée en ce qu**'elle comprend au moins un coupleur choisi parmi les métaphénylène diamines, les méta-aminophénols, les métadiphénols et les coupleurs hétérocycliques ou leurs sels d'addition avec un acide ou avec un agent alcalin.

17. Composition selon la revendication précédente, **caractérisée en ce que** le ou les coupleurs représentent de 0,0001 à 10 % en poids, plus particulièrement 0,005 à 5 % en poids, du poids total de la composition tinctoriale.

18. Composition, **caractérisée en ce qu**'elle comprend la composition selon l'une des revendications 1 à 12 et 14 à 17, et au moins un agent oxydant.

19. Composition selon la revendication précédente, **caractérisée en ce que** l'agent oxydant est choisi parmi le peroxyde d'hydrogène, le peroxyde d'urée, les bromates de métaux alcalins, les persels tels que les perborates et persulfates, et les enzymes telles que les peroxydases et les oxydo-réductases à deux ou quatre électrons, et de préférence le peroxyde d'hydrogène.

20. Procédé mettant en oeuvre une composition comprenant dans un milieu cosmétiquement acceptable, au moins un colorant fluorescent soluble dans ledit milieu, au moins un polymère conditionneur insoluble dans ledit milieu, choisi parmi les polyorganosiloxanes ne portant pas de groupement aminé, pour la coloration avec effet éclaircissant de cheveux présentant une hauteur de ton inférieur ou égale à 6.

21. Procédé selon la revendication 20, **caractérisé en ce que** le colorant fluorescent conduit à un maximum de réflectance se situant dans la gamme de longueur d'onde allant de 500 à 650 nanomètres, et de préférence dans la gamme de longueur d'onde allant de 550 à 620 nanomètres.

22. Procédé selon l'une quelconque des revendications 20 ou 21, **caractérisé en ce que** le colorant fluorescent est choisi parmi les composés fluorescents appartenant aux familles suivantes : les naphtalimides ; les coumarines cationiques ou non : les xanthénodiquinolizines ; les azaxanthènes ; les naphtolactames ; les azlactones ; les oxazines ; les thiazines ; les dioxazines ; les colorants fluorescents monocationiques ou polycationiques de type azoïque, azométhinique, ou méthinique, seuls ou en mélanges.

23. Procédé selon l'une quelconque des revendications 20 à 22, **caractérisé en ce que** le colorant fluorescent est choisi dans le groupe formé par les colorants de structures suivantes : 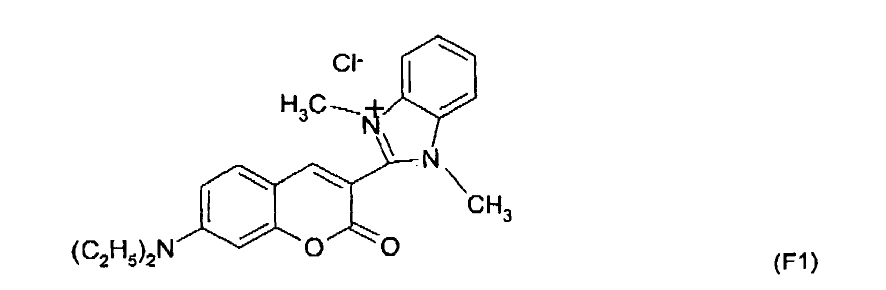 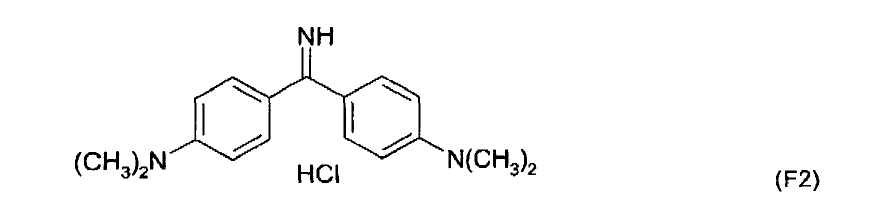 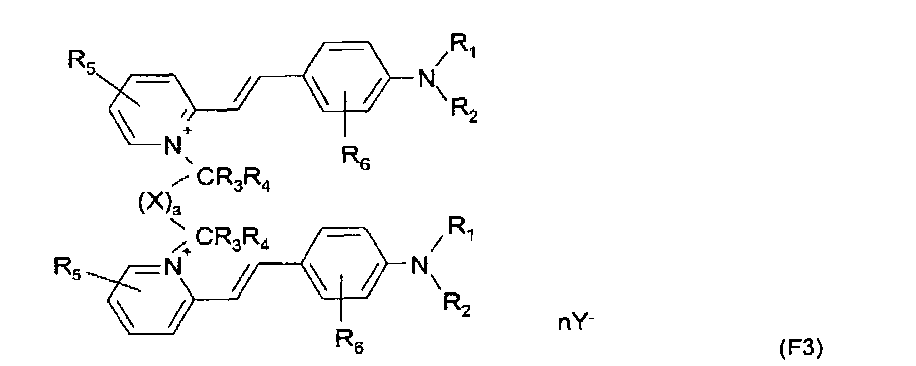 dans laquelle :
R₁, R₂, identiques ou différents, représentent :
• un atome d'hydrogène ;
• un radical alkyle, linéaire ou ramifié, comprenant 1 à 10 atomes de carbone, de préférence de 1 à 4 atomes de carbone, éventuellement interrompu et/ou substitué par au moins un hétéroatome et/ou groupement comprenant au moins un hétéroatome et/ou substitué par au moins un atome d'halogène;
• un radical aryle ou arylalkyle, le groupement aryle ayant 6 atomes de carbone et le radical alkyle ayant 1 à 4 atomes de carbone ; le radical aryle étant éventuellement substitué par un ou plusieurs radicaux alkyles linéaires ou ramifiés comprenant 1 à 4 atomes de carbone éventuellement interrompus et/ou substitués par au moins un hétéroatome et/ou groupement comprenant au moins un hétéroatome et/ou substitué par au moins un atome d'halogène ;
• R₁ et R₂ peuvent éventuellement être reliés de manière à former un hétérocycle avec l'atome d'azote et comprendre un ou plusieurs autres hétéroatomes, l'hétérocycle étant éventuellement substitué par au moins un radical alkyle linéaire ou ramifié, comprenant de préférence de 1 à 4 atomes de carbone et étant éventuellement interrompu et/ou substitué par au moins un hétéroatome et/ou groupement comprenant au moins un hétéroatome et/ou substitué par au moins un atome d'halogène ;
• R₁ ou R₂ peut éventuellement être engagé dans un hétérocycle comprenant l'atome d'azote et l'un des atomes de carbone du groupement phényle portant ledit atome d'azote ;
R₃, R₄, identiques ou non, représentent un atome d'hydrogène, un radical alkyle comprenant 1 à 4 atomes de carbone ;
R₅, identiques ou non, représentent un atome d'hydrogène, un atome d'halogène, un radical alkyle linéaire ou ramifié comprenant 1 à 4 atomes de carbone éventuellement interrompu par au moins un hétéroatome ;
R₆, identiques ou non, représentent un atome d'hydrogène ; un atome d'halogène ; un radical alkyle linéaire ou ramifié comprenant 1 à 4 atomes de carbone, éventuellement substitué et/ou interrompu par au moins un hétéroatome et/ou groupement portant au moins un hétéroatome et/ou substitué par au moins un atome d'halogène ;
X représente :
• un radical alkyle, linéaire ou ramifié comprenant 1 à 14 atomes de carbone, ou alcényle comprenant 2 à 14 atomes de carbone, éventuellement interrompu et/ou substitué par au moins un hétéroatome et/ou groupement comprenant au moins un hétéroatome et/ou substitué par au moins un atome d'halogéne ;
• un radical hétérocyclique comprenant 5 ou 6 chaînons, éventuellement substitué par au moins un radical alkyle linéaire ou ramifié comprenant 1 à 14 atomes de carbone, éventuellement substitué par au moins un hétéroatome ; par au moins un radical aminoalkyle, linéaire ou ramifié, comprenant 1 à 4 atomes de carbone, éventuellement substitué par au moins un hétéroatome ; par au moins un atome d'halogène :
• un radical aromatique ou diaromatique condensé ou non, séparé ou non par un radical alkyle comprenant 1 à 4 atomes de carbone, le ou les radicaux aryles étant éventuellement substitués par au moins un atome d'halogène ou par au moins un radical alkyle comprenant 1 à 10 atomes de carbone éventuellement substitué et/ou interrompu par au moins un hétéroatome et/ou groupement portant au moins un hétéroatome;
• un radical dicarbonyle ;
• le groupement X pouvant porter une ou plusieurs charges cationiques ;
a étant égal à 0 ou 1 ;
Y⁻, identiques ou non, représentant un anion organique ou minéral ;
n étant un nombre entier au moins égal à 2 et au plus égal au nombre de charges cationiques présentes dans le composé fluorescent ; 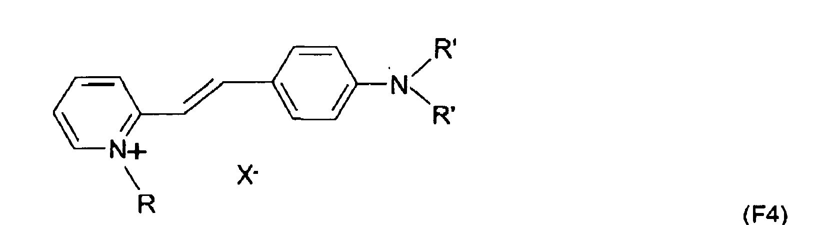 formule dans laquelle R représente un radical méthyle ou éthyle; R' représente un radical méthyle, X- un anion du type chlorure, iodure, sulfate, méthosulfate, acétate, perchlorate..

24. Procédé selon l'une quelconque des revendications 20 à 23, **caractérisé en ce que** les cheveux sont des fibres kératiniques pigmentées ou colorées artificiellement.

25. Procédé selon l'une quelconque des revendications 20 à 24, **caractérisé en ce que** les cheveux présentent une hauteur de ton inférieure ou égale à 4.

26. Procédé pour colorer avec un effet éclaircissant selon l'une quelconque des revendications 20 à 25, **caractérisé en ce que** l'on met en oeuvre les étapes suivantes :
a) on applique sur lesdites fibres une composition telle que définie selon l'une quelconque des revendications 20 à 23, pendant un temps suffisant pour développer la coloration et l'éclaircissement désirés,
b) on rince éventuellement les fibres,
c) éventuellement on lave au shampooing et on rince les fibres,
d) on sèche ou on laisse sécher les fibres.

27. Procédé selon la revendication 26, **caractérisé en ce qu**'il comporte une étape préliminaire consistant à stocker sous forme séparée, d'une part, une composition selon l'une des revendications 20 à 23, et d'autre part, une composition renfermant, dans un milieu cosmétiquement acceptable, au moins un agent oxydant, puis à procéder à leur mélange au moment de l'emploi avant d'appliquer ce mélange sur les fibres pendant un temps suffisant pour développer la coloration désirée, après quoi on les rince, on les lave éventuellement au shampooing, on les rince à nouveau et on les sèche.

28. Procédé pour colorer avec un effet éclaircissant une peau foncée, **caractérisé en ce que** l'on applique sur la peau une composition selon l'une quelconque des revendications 1 à 12, puis on sèche ou on laisse sécher la peau.

29. Dispositif à plusieurs compartiments pour la teinture et l'éclaircissement des fibres kératiniques, comprenant au moins un compartiment renfermant une composition selon l'une des revendications 1 à 12 et 14 à 17, et au moins un autre compartiment renfermant une composition renfermant au moins un agent oxydant.

## Claims

1. Composition, **characterized in that** it comprises, in a cosmetically acceptable medium, at least one dye fluorescent in the orange range that is soluble in the said medium and at least one conditioning polymer that is insoluble in the said medium, chosen from polyorganosiloxanes which do not bear an amine group; the composition not comprising, as fluorescent agent, 2-[2-(4-dialkylamino)phenylethenyl]-1-alkylpyridinium in which the alkyl radical of the pyridinium nucleus represents a methyl or ethyl radical, that of the benzene nucleus represents a methyl radical and in which the counterion is a halide.

2. Composition according to the preceding claim, **characterized in that** the fluorescent dye leads to a reflectance maximum that is in the wavelength range from 500 to 650 nanometres and preferably in the wavelength range from 550 to 620 nanometres.

3. Composition according to either of the preceding claims, **characterized in that** the fluorescent dye is chosen from the fluorescent dyes belonging to the following families: naphthalimides; cationic or non-cationic coumarins; xanthenodiquinolizines; azaxanthenes; naphtholactams; azlactones; oxazines; thiazines; dioxazines; polycationic fluorescent dyes of azo, azomethine or methine type, alone or as mixtures.

4. Composition according to any one of the preceding claims, **characterized in that** the fluorescent compound has the following formula: 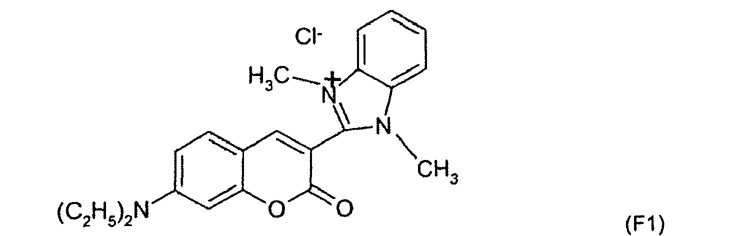 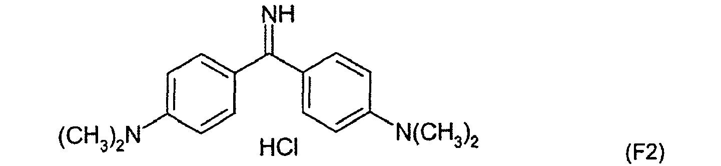 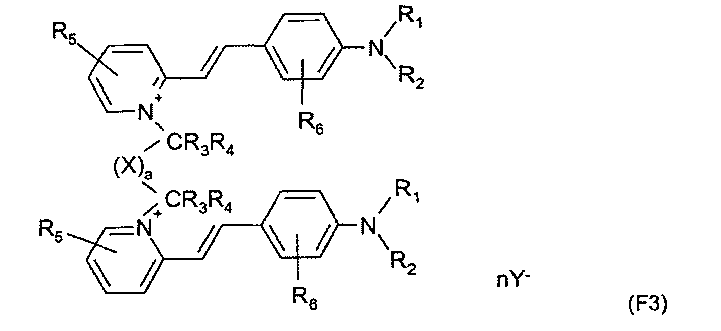 in which:
R₁ and R₂, which may be identical or different, represent:
• a hydrogen atom;
• a linear or branched alkyl radical containing 1 to 10 carbon atoms and preferably from 1 to 4 carbon atoms, optionally interrupted and/or substituted with at least one hetero atom and/or group comprising at least one hetero atom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one hetero atom and/or group comprising at least one hetero atom and/or substituted with at least one halogen atom;
• R₁ and R₂ may optionally be linked so as to form a heterocycle with the nitrogen atom and may comprise one or more other hetero atoms, the heterocycle optionally being substituted with at least one linear or branched alkyl radical preferably containing from 1 to 4 carbon atoms and optionally being interrupted and/or substituted with at least one hetero atom and/or group comprising at least one hetero atom and/or substituted with at least one halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one hetero atom;
R₆, which may be identical or different, represent a hydrogen atom; a halogen atom; a linear or branched alkyl radical containing 1 to 4 carbon atoms, optionally substituted and/or interrupted with at least one hetero atom and/or group bearing at least one hetero atom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one hetero atom and/or group containing at least one hetero atom and/or substituted with at least one halogen atom;
• a 5- or 6-membered heterocyclic radical optionally substituted with at least one linear or branched alkyl radical containing 1 to 14 carbon atoms, optionally substituted with at least one hetero atom; with at least one linear or branched aminoalkyl radical containing 1 to 4 carbon atoms, optionally substituted with at least one hetero atom; with at least one halogen atom;
• a fused or non-fused aromatic or diaromatic radical, optionally separated with an alkyl radical containing 1 to 4 carbon atoms, the aryl radical(s) optionally being substituted with at least one halogen atom or with at least one alkyl radical containing 1 to 10 carbon atoms optionally substituted and/or interrupted with at least one hetero atom and/or group bearing at least one hetero atom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound.

5. Composition according to any one of the preceding claims, **characterized in that** the fluorescent dye(s) is(are) present in a weight concentration of between 0.01% and 20% by weight, more particularly between 0.05% and 10% by weight and preferably between 0.1% and 5% by weight relative to the total weight of the composition.

6. Composition according to any one of the preceding claims, **characterized in that** the insoluble conditioning polymer is chosen from silicones in the form of oils, waxes, resins or gums, for instance cyclic volatile silicones containing from 3 to 7 silicon atoms, cyclocopolymers, linear volatile silicones containing from 2 to 9 silicon atoms, non-volatile silicones of the polyalkylsiloxane, polyarylsiloxane, polyalkylarylsiloxane or polyorganosiloxane type modified with organofunctional groups, for instance polyethyleneoxy and/or polypropyleneoxy groups optionally comprising alkyl groups, thiol groups, alkoxylated groups, hydroxylated groups, acyloxyalkyl groups, anionic groups of the carboxylic, sulphonate or thiosulphate type; grafted silicones comprising a polysiloxane portion and a portion consisting of a non-silicone organic chain, and polydiorganosiloxane, organopolysiloxane or trimethylsiloxysilicate resins, alone or as a mixture.

7. Composition according to one of the preceding claims, **characterized in that** the content of insoluble conditioning polymer represents 0.01% to 20% by weight relative to the weight of the composition and more particularly 0.1% to 10% by weight relative to the same reference.

8. Composition according to one of the preceding claims, **characterized in that** it comprises at least one nonionic, anionic or amphoteric surfactant.

9. Composition according to the preceding claim, **characterized in that** the surfactant content represents 0.01% to 30% by weight relative to the total weight of the composition.

10. Composition according to any one of the preceding claims, **characterized in that** it also comprises at least one non-fluorescent additional direct dye of nonionic, cationic or anionic nature.

11. Composition according to Claim 10, **characterized in that** the additional direct dyes are chosen from nitrobenzene dyes, azo dyes, anthraquinone dyes, naphthoquinone dyes, benzoquinone dyes, phenothiazine dyes, indigoid dyes, xanthene dyes, phenanthridine dyes, phthalocyanin dyes and triarylmethane-based dyes, or mixtures thereof.

12. Composition according to either of Claims 10 and 11, **characterized in that** the additional direct dye(s) represent (s) from 0.0005% to 12% by weight and preferably from 0.005% to 6% by weight relative to the total weight of the composition.

13. Composition according to any one of the preceding claims, **characterized in that** it is in the form of a lightening dyeing shampoo.

14. Composition according to any one of the preceding claims, **characterized in that** it comprises at least one oxidation base chosen from para-phenylenediamines, bis(phenyl)alkylenediamines, para-aminophenols, ortho-aminophenols and heterocyclic bases, or the addition salts thereof with an acid or with an alkaline agent.

15. Composition according to the preceding claim, **characterized in that** the oxidation base(s) represent(s) 0.0005% to 12% by weight and more particularly 0.005% to 6% by weight relative to the total weight of the composition.

16. Composition according to either of Claims 14 and 15, **characterized in that** it comprises at least one coupler chosen from meta-phenylenediamines, meta-aminophenols, meta-diphenols and heterocyclic couplers, or the addition salts thereof with an acid or with an alkaline agent.

17. Composition according to the preceding claim, **characterized in that** the coupler(s) represent(s) from 0.0001% to 10% by weight and more particularly 0.005% to 5% by weight relative to the total weight of the dye composition.

18. Composition, **characterized in that** it comprises the composition according to one of Claims 1 to 12 and 14 to 17, and at least one oxidizing agent.

19. Composition according to the preceding claim, **characterized in that** the oxidizing agent is chosen from hydrogen peroxide, urea peroxide, alkali metal bromates, persalts such as perborates and persulphates, and enzymes such as peroxidases and two-electron or four-electron oxidoreductases, and preferably hydrogen peroxide.

20. Process employing a composition comprising, in a cosmetically acceptable medium, at least one fluorescent dye that is soluble in the said medium and at least one conditioning polymer that is insoluble in the said medium, chosen from polyorganosiloxanes not bearing an amino group, to dye hair having a tone height of less than or equal to 6 with a lightening effect.

21. Process according to Claim 20, **characterized in that** the fluorescent dye gives a reflectance maximum that is in the wavelength range from 500 to 650 nanometres and preferably in the wavelength range from 550 to 620 nanometres.

22. Process according to either of Claims 20 and 21, **characterized in that** the fluorescent dye is chosen from fluorescent compounds belonging to the following families: naphthalimides; cationic or non-cationic coumarins; xanthenodiquinolizines; azaxanthenes; naphtholactams; azlactones; oxazines; thiazines; dioxazines; monocationic and polycationic fluorescent dyes of azo, azomethine or methine type, alone or as mixtures.

23. Process according to any one of Claims 20 to 22, **characterized in that** the fluorescent dye is chosen from the group formed by the dyes having the following structures: 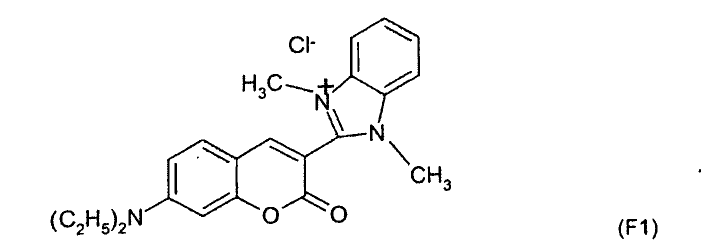 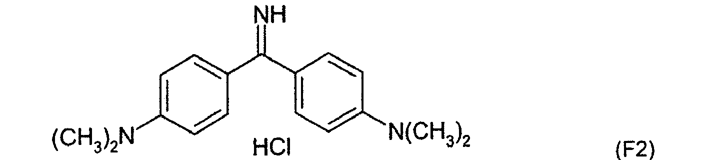 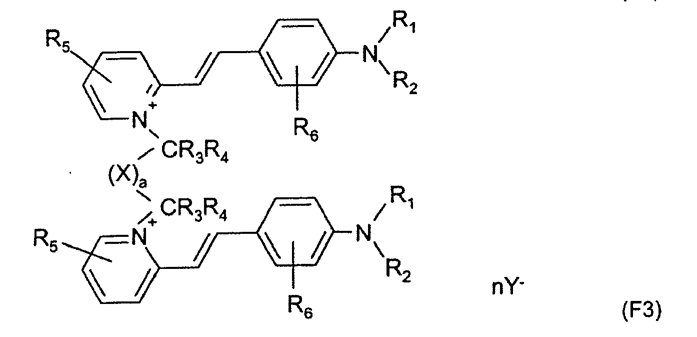 in which:
R₁ and R₂, which may be identical or different, represent:
• a hydrogen atom;
• a linear or branched alkyl radical containing 1 to 10 carbon atoms and preferably from 1 to 4 carbon atoms, optionally interrupted and/or substituted with at least one hetero atom and/or group comprising at least one hetero atom and/or substituted with at least one halogen atom;
• an aryl or arylalkyl radical, the aryl group containing 6 carbon atoms and the alkyl radical containing 1 to 4 carbon atoms; the aryl radical optionally being substituted with one or more linear or branched alkyl radicals comprising 1 to 4 carbon atoms optionally interrupted and/or substituted with at least one hetero atom and/or group comprising at least one hetero atom and/or substituted with at least one halogen atom;
• R₁ and R₂ may optionally be linked so as to form a heterocycle with the nitrogen atom and may comprise one or more other hetero atoms, the heterocycle optionally being substituted with at least one linear or branched alkyl radical preferably containing from 1 to 4 carbon atoms and optionally being interrupted and/or substituted with at least one hetero atom and/or group comprising at least one hetero atom and/or substituted with at least one halogen atom;
• R₁ or R₂ may optionally be engaged in a heterocycle comprising the nitrogen atom and one of the carbon atoms of the phenyl group bearing the said nitrogen atom;
R₃ and R₄, which may be identical or different, represent a hydrogen atom or an alkyl radical containing 1 to 4 carbon atoms;
R₅, which may be identical or different, represent a hydrogen atom, a halogen atom or a linear or branched alkyl radical containing 1 to 4 carbon atoms, optionally interrupted with at least one hetero atom;
R₆, which may be identical or different, represent a hydrogen atom; a halogen atom; a linear or branched alkyl radical containing 1 to 4 carbon atoms, optionally substituted and/or interrupted with at least one hetero atom and/or group bearing at least one hetero atom and/or substituted with at least one halogen atom;
X represents:
• a linear or branched alkyl radical containing 1 to 14 carbon atoms or an alkenyl radical containing 2 to 14 carbon atoms, optionally interrupted and/or substituted with at least one hetero atom and/or group containing at least one hetero atom and/or substituted with at least one halogen atom;
• a 5- or 6-membered heterocyclic radical optionally substituted with at least one linear or branched alkyl radical containing 1 to 14 carbon atoms, optionally substituted with at least one hetero atom; with at least one linear or branched aminoalkyl radical containing 1 to 4 carbon atoms, optionally substituted with at least one hetero atom; with at least one halogen atom;
• a fused or non-fused aromatic or diaromatic radical, optionally separated with an alkyl radical containing 1 to 4 carbon atoms, the aryl radical(s) optionally being substituted with at least one halogen atom or with at least one alkyl radical containing 1 to 10 carbon atoms optionally substituted and/or interrupted with at least one hetero atom and/or group bearing at least one hetero atom;
• a dicarbonyl radical;
• the group X possibly bearing one or more cationic charges;
a being equal to 0 or 1;
Y⁻, which may be identical or different, representing an organic or mineral anion;
n being an integer at least equal to 2 and at most equal to the number of cationic charges present in the fluorescent compound; 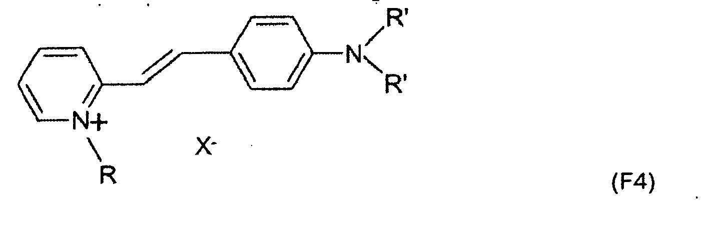 in which formula R represents a methyl or ethyl radical; R' represents a methyl radical and X⁻represents an anion such as chloride, iodide, sulphate, methasulphate, acetate or perchlorate.

24. Process according to any one of Claims 20 to 23, **characterized in that** the hair is artificially coloured or pigmented keratin fibres.

25. Process according to any one of Claims 20 to 24, **characterized in that** the hair has a tone height of less than or equal to 4.

26. Process for dyeing with a lightening effect according to any one of Claims 20 to 25, **characterized in that** the following steps are performed:
a) a composition as defined according to any one of Claims 20 to 23 is applied to the said fibres, for a time that is sufficient to develop the desired coloration and lightening,
b) the fibres are optionally rinsed,
c) the fibres are optionally washed with shampoo and rinsed,
d) the fibres are dried or are left to dry.

27. Process according to Claim 26, **characterized in that** it comprises a preliminary step that consists in separately storing, on the one hand, a composition according to one of Claims 20 to 23, and, on the other hand, a composition containing, in a cosmetically acceptable medium, at least one oxidizing agent, and then in mixing them together at the time of use, followed by applying this mixture to the fibres for a time that is sufficient to develop the desired coloration, after which the fibres are rinsed, optionally washed with shampoo, rinsed again and dried.

28. Process for colouring dark skin with a lightening effect, **characterized in that** a composition according to any one of Claims 1 to 12 is applied to the skin and the skin is then dried or is left to dry.

29. Multi-compartment device for dyeing and lightening keratin fibres, comprising at least one compartment containing a composition according to one of Claims 1 to 12 and 14 to 17, and at least one other compartment containing a composition containing at least one oxidizing agent.

## Patentansprüche

1. Zusammensetzung, **dadurch gekennzeichnet, dass** sie in einem kosmetisch akzeptablen Medium mindestens einen in dem Medium löslichen, im Orangebereich fluoreszierenden Farbstoff und mindestens ein in dem Medium unlösliches konditionierendes Polymer enthält, das unter den Polyorganosiloxanen ausgewählt ist, die keine aminierte Gruppe aufweisen, wobei die Zusammensetzung als fluoreszierenden Stoff kein 2-[2-(4-Dialkylamino)phenyl-ethenyl)-1-alkylpyridinium enthält, bei dem die Alkylgruppe des Pyridiniumrings eine Methylgruppe oder eine Ethylgruppe bedeutet und die Alkylgruppe des Benzolkerns eine Methylgruppe bedeutet und bei dem das Gegenion ein Halogenid ist.

2. Zusammensetzung nach dem vorhergehenden Anspruch, **dadurch gekennzeichnet, dass** der fluoreszierende Farbstoff zu einem maximalen Reflexionsgrad im Wellenlängenbereich von 500 bis 650 nm und vorzugsweise im Wellenlängenbereich von 550 bis 620 nm führt.

3. Zusammensetzung nach einem der vorhergehenden Ansprüche, **dadurch gekennzeichnet, dass** der fluoreszierende Farbstoff unter den fluoreszierenden Farbstoffen der folgenden Gruppen ausgewählt ist: Naphthalimiden; kationischen oder nicht kationischen Cumarinen; Xanthenodichinolizinen; Azaxanthenen; Naphtholactamen; Azlactonen; Oxazinen; Thiazinen; Dioxazinen; polykationischen fluoreszierenden Farbstoffen vom Azotyp, Azomethintyp oder Methintyp, wobei diese Farbstoffe einzeln oder im Gemisch vorliegen.

4. Zusammensetzung nach einem der vorhergehenden Ansprüche, **dadurch gekennzeichnet, dass** die fluoreszierende Verbindung die folgende Formel aufweist: 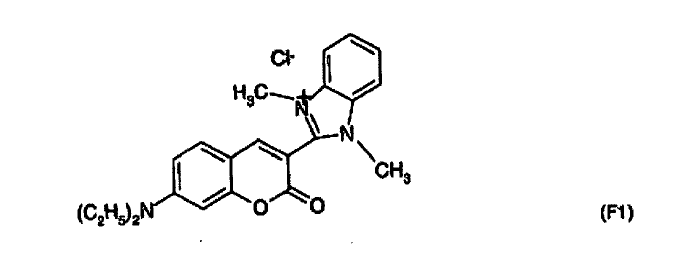 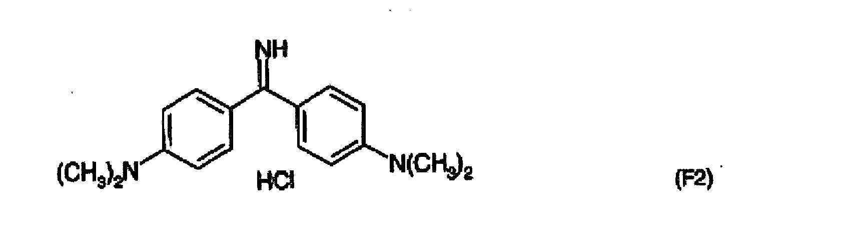 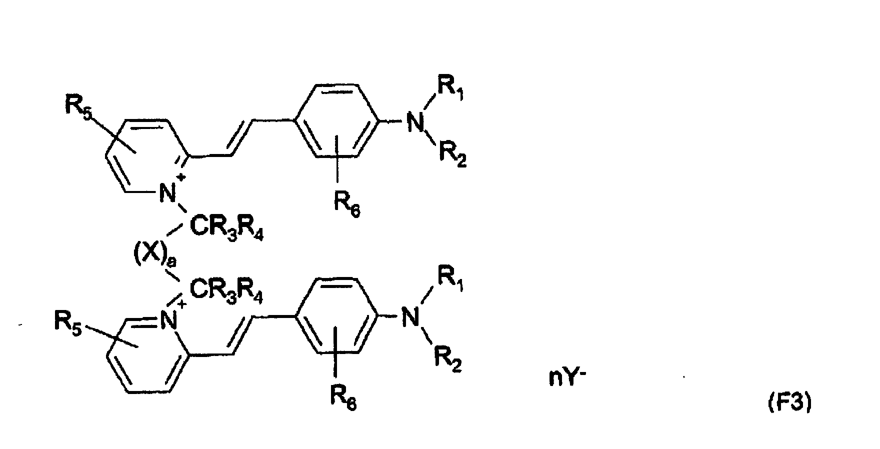 worin bedeuten:
die Gruppen R₁ und R₂, die gleich oder verschieden sind:
. ein Wasserstoffatom;
. eine geradkettige oder verzweigte Alkylgruppe mit 1 bis 10 Kohlenstoffatomen und vorzugsweise 1 bis 4 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
. eine Aryl- oder Arylalkylgruppe, wobei die Arylgruppe 6 Kohlenstoffatome und die Alkylgruppe 1 bis 4 Kohlenstoffatome besitzt, wobei die Arylgruppe gegebenenfalls mit einer oder mehreren geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert sind und/oder mit mindestens einem Halogenatom substituiert sind;
. die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
. die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält;
die Gruppen R₃ und R₄, die identisch oder verschieden sind, ein Wasserstoffatom oder eine Alkylgruppe mit 1 bis 4 Kohlenstoffatomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/oder einer Gruppe, die mindestens ein Heteroatom enthält, substituiert und/ oder durch mindestens ein Heteroatom und/oder eine Gruppe, die mindestens ein Heteroatom enthält, unterbrochen ist und/oder mit mindestens einem Halogenatom substituiert ist;
X bedeutet:
. eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/ oder mit mindestens einem Heteroatom und/oder einer Gruppe, die mindestens ein Heteroatom enthält, substituiert und/oder mit mindestens einem Halogenatom substituiert sind;
. eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
· eine aromatische Gruppe oder eine kondensierte oder nicht kondensierte, diaromatische Gruppe, die gegebenenfalls durch eine Alkylgruppe mit 1 bis 4 Kohlenstoffatomen separiert wird, wobei die Arylgruppe(n) gegebenenfalls mit mindestens einem Halogenatom oder mit mindestens einer Alkylgruppe mit 1 bis 10 Kohlenstoffatomen substituiert sind, die gegebenenfalls mit mindestens einem Heteroatom und/oder eine Gruppe, die mindestens ein Heteroatom enthält, substituiert und/oder durch mindestens ein Heteroatom und/oder eine Gruppe, die mindestens ein Heteroatom enthält, unterbrochen ist;
· eine Dicarbonylgruppe;
. wobei die Gruppe X eine oder mehrere kationische Ladungen umfassen kann;
wobei a 0 oder 1 beträgt;
die Gruppen Y⁻, die gleich oder verschieden sind, ein organisches oder anorganisches Anion;
wobei n eine ganze Zahl von mindestens 2 und höchstens der Anzahl der in der fluoreszierenden Verbindung vorliegenden kationischen Ladungen ist.

5. Zusammensetzung nach einem der vorhergehenden Ansprüche, **dadurch gekennzeichnet, dass** der oder die fluoreszierende(n) Farbstoff(e) in einer Konzentration von 0,01 bis 20 Gew.-%, insbesondere 0,05 bis 10 Gew.-% und vorzugsweise 0,1 bis 5 Gew.%, bezogen auf das Gesamtgewicht der Zusammensetzung, enthalten sind.

6. Zusammensetzung nach einem der vorhergehenden Ansprüche, **dadurch gekennzeichnet, dass** das unlösliche konditionierende Polymer ausgewählt ist unter in Form von Ölen, Wachsen, Harzen oder Gummis vorliegenden Siliconen, wie cyclischen flüchtigen Siliconen mit 3 bis 7 Siliciumatomen, Cyclocopolymeren, linearen flüchtigen Siliconen mit 2 bis 9 Siliciumatomen, nicht flüchtigen Siliconen vom Typ der Polyalkylsiloxane, Polyarylsiloxane, Polyalkylarylsiloxane, mit organofunktionellen Gruppen modifizierten Polyorganosiloxanen, beispielsweise mit Polyethylenoxy- und/oder Polypropylenoxygruppen, die gegebenenfalls Alkylgruppen, Thiolgruppen, Alkoxygruppen, Hydroxygruppen, Acyloxyalkylgruppen, anionische Gruppen von Carboxytyp, Sulfonattyp, Thiosulfattyp aufweisen; gepfropften Siliconen, die einen Siloxanbereich und einen Bereich enthalten, der aus einer nicht siliconhaltigen, organischen Kette besteht, Polydiorganosiloxanharzen, Organopolysiloxanen, Trimethylsiloxysilicat oder deren Gemischen.

7. Zusammensetzung nach einem der vorhergehenden Ansprüche, **dadurch gekennzeichnet, dass** der Mengenanteil des unlöslichen konditionierenden Polymers 0,01 bis 20 Gew.-%, bezogen auf das Gesamtgewicht der Zusammensetzung, und insbesondere 0,1 bis 10 Gew.-%, bezogen auf das Gesamtgewicht der Zusammensetzung, ausmacht.

8. Zusammensetzung nach einem der vorhergehenden Ansprüche, **dadurch gekennzeichnet, dass** sie mindestens einen nichtionischen, anionischen oder amphoteren grenzflächenaktiven Stoff enthält.

9. Zusammensetzung nach dem vorhergehenden Anspruch, **dadurch gekennzeichnet, dass** der Mengenanteil des grenzflächenaktiven Stoffes im Bereich von 0,01 bis 30 Gew.-%, bezogen auf das Gesamtgewicht der Zusammensetzung, liegt.

10. Zusammensetzung nach einem der vorhergehenden Ansprüche, **dadurch gekennzeichnet, dass** sie ferner mindestens einen zusätzlichen, nicht fluoreszierenden Direktfarbstoff vom nichtionischen, kationischen oder anionischen Typ enthält.

11. Zusammensetzung nach Anspruch 10, **dadurch gekennzeichnet, dass** die zusätzlichen Direktfarbstoffe unter den nitrierten Benzolfarbstoffen, Azofarbstoffen, Anthrachinon-Farbstoffen, Naphthochinon-Farbstoffen, Benzochinon-Farbstoffen, Phenotiazin-Farbstoffen, Indigoiden, Xanthen-Farbstoffen, Phenanthridin-Farbstoffen, Phthalocyaninen sowie den von Triarylmethan abgeleiteten Farbstoffen oder deren Gemischen ausgewählt sind.

12. Zusammensetzung nach einem der Ansprüche 10 oder 11, **dadurch gekennzeichnet, dass** der oder die zusätzliche(n) Direktfarbstoff(e) 0,0005 bis 12 Gew.-% und vorzugsweise 0,005 bis 6 Gew.-% des Gesamtgewichts der Zusammensetzung ausmachen.

13. Zusammensetzung nach einem der vorhergehenden Ansprüche, **dadurch gekennzeichnet, dass** sie in Form eines aufhellenden und färbenden Haarwaschmittels vorliegt.

14. Zusammensetzung nach einem der vorhergehenden Ansprüche, **dadurch gekennzeichnet, dass** sie ferner mindestens eine Oxidationsbase enthält, die unter den p-Phenylendiaminen, Bisphenylalkylendiaminen, p-Aminophenolen, o-Aminophenolen und den heterocyclischen Basen sowie den Additionssalzen dieser Verbindungen mit einer Säure oder einem alkalischen Stoff ausgewählt ist.

15. Zusammensetzung nach dem vorhergehenden Anspruch, **dadurch gekennzeichnet, dass** die Oxidationsbase(n) in Konzentrationen von 0,0005 bis 12 Gew.-% und insbesondere 0,005 bis 6 Gew.-%, bezogen auf das Gesamtgewicht der Zusammensetzung, enthalten sind.

16. Zusammensetzung nach einem der Ansprüche 14 oder 15, **dadurch gekennzeichnet, dass** sie ferner mindestens einen Kuppler enthält, der unter den m-Phenylendiaminen, m-Aminophenolen, m-Dihydroxybenzolen, heterocyclischen Kupplern oder den Additionssalzen dieser Verbindungen mit einer Säure oder einem alkalischen Stoff ausgewählt ist.

17. Zusammensetzung nach dem vorhergehenden Anspruch, **dadurch gekennzeichnet, dass** der oder die Kuppler in Mengenanteilen von 0,0001 bis 10 Gew.-% und insbesondere 0,005 bis 5 Gew.-%, bezogen auf das Gesamtgewicht der Zusammensetzung, enthalten sind.

18. Zusammensetzung, **dadurch gekennzeichnet, dass** sie die Zusammensetzung nach einem der Ansprüche 1 bis 12 und 14 bis 17 und mindestens ein Oxidationsmittel umfasst.

19. Zusammensetzung nach dem vorhergehenden Anspruch, **dadurch gekennzeichnet, dass** das Oxidationsmittel unter Wasserstoffperoxid, Harnstoffperoxid, Alkalimetallbromaten, Salzen von Persäuren, wie Perboraten und Persulfaten, und Enzymen, wie Peroxidasen und Oxidoreduktasen (2 oder 4 Elektronen), und vorzugsweise Wasserstoffperoxid ausgewählt ist.

20. Verfahren unter Verwendung einer Zusammensetzung, die in einem kosmetisch akzeptablen Medium mindestens einen in dem Medium löslichen, fluoreszierenden Farbstoff und mindestens ein in dem Medium unlösliches, konditionierendes Polymer enthält, das unter den Polyorganosiloxanen ausgewählt ist, die keine aminierte Gruppe aufweisen, zum Färben von Haaren, die einen Farbton von 6 oder darunter aufweisen, mit aufhellender Wirkung.

21. Verfahren nach Anspruch 20, **dadurch gekennzeichnet, dass** der fluoreszierende Farbstoff zu einem maximalen Reflexionsgrad im Wellenlängenbereich von 500 bis 650 nm und vorzugsweise im Wellenlängenbereich von 550 bis 620 nm führt.

22. Verwendung nach einem der Ansprüche 20 oder 21, **dadurch gekennzeichnet, dass** der fluoreszierende Farbstoff unter den fluoreszierenden Farbstoffen aus den folgenden Gruppen ausgewählt ist: Naphthalimiden; kationischen oder nicht kationischen Cumarinen; Xanthenodichinolizinen; Azaxanthenen; Naphtholactamen; Azlactonen; Oxazinen; Thiazinen; Dioxazinen; mono- oder polykationischen fluoreszierenden Farbstoffen vom Azotyp, Azomethintyp oder Methintyp, wobei diese Farbstoffe einzeln oder im Gemisch vorliegen.

23. Verfahren nach einem der Ansprüche 20 bis 22, **dadurch gekennzeichnet, dass** der fluoreszierende Farbstoff unter den Farbstoffen der folgenden Strukturen ausgewählt ist: 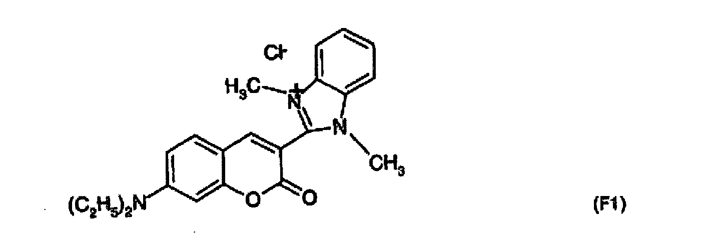 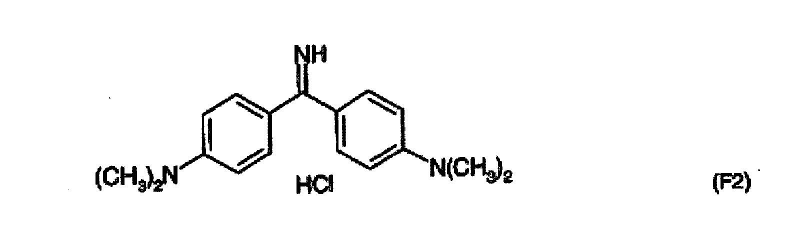 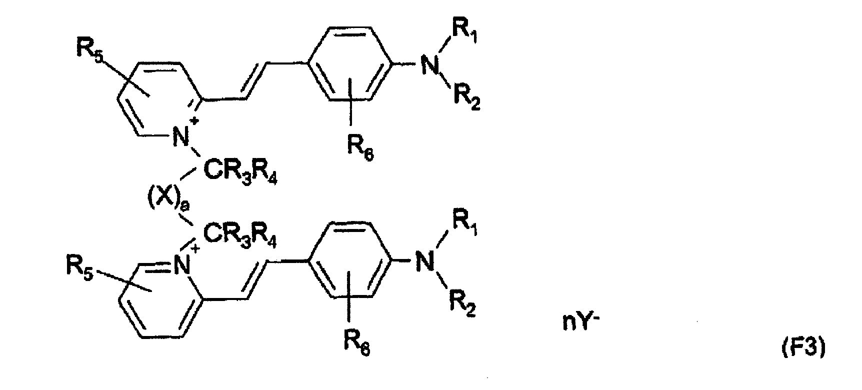 worin bedeuten:
die Gruppen R₁ und R₂, die gleich oder verschieden sind:
. ein Wasserstoffatom;
. eine geradkettige oder verzweigte Alkylgruppe mit 1 bis 10 Kohlenstoffatomen und vorzugsweise 1 bis 4 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· eine Aryl- oder Arylalkylgruppe, wobei die Arylgruppe 6 Kohlenstoffatome und die Alkylgruppe 1 bis 4 Kohlenstoffatome besitzt, wobei die Arylgruppe gegebenenfalls mit einer oder mehreren geradkettigen oder verzweigten Alkylgruppen mit 1 bis 4 Kohlenstoffatomen substituiert ist, die gegebenenfalls durch mindestens ein Heteroatom und/oder eine Gruppe, die mindestens ein Heteroatom enthält, unterbrochen sind und/oder mit mindestens einem Heteroatom und/oder einer Gruppe, die mindestens ein Heteroatom enthält, substituiert sind und/oder mit mindestens einem Halogenatom substituiert sind;
. die Gruppen R₁ und R₂ können gegebenenfalls so verbunden sein, dass sie mit dem Stickstoffatom einen Heterocyclus bilden, wobei ein oder mehrere weitere Heteroatome enthalten sein können, wobei der Heterocyclus gegebenenfalls mit mindestens einer geradkettigen oder verzweigten Alkylgruppe substituiert ist, die vorzugsweise 1 bis 4 Kohlenstoffatome enthält und gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert ist und/oder mit mindestens einem Halogenatom substituiert ist;
· die Gruppe R₁ oder R₂ kann gegebenenfalls in einen Heterocyclus eingebunden sein, der das Stickstoffatom und ein Kohlenstoffatom des Phenylrings, der das Stickstoffatom trägt, enthält;
die Gruppen R₃ und R₄, die identisch oder verschieden sind, ein Wasserstoffatom oder eine Alkylgruppe mit 1 bis 4 Kohlenstoffatomen;
die Gruppen R₅, die gleich oder verschieden sind, ein Wasserstoffatom; ein Halogenatom; eine geradkettige oder verzweigte Alkylgruppe mit 1 bis 4 Kohlenstoffatomen, die gegebenenfalls durch mindestens ein Heteroatom unterbrochen ist;
die Gruppen R₆, die gleich oder verschieden sind, ein Wasserstoffatom; ein Halogenatom; eine geradkettige oder verzweigte Alkylgruppe mit 1 bis 4 Kohlenstoffatomen, die gegebenenfalls mit mindestens einem Heteroatom und/ oder einer Gruppe, die mindestens ein Heteroatom enthält, substituiert und/oder durch mindestens ein Heteroatom und/oder eine Gruppe, die mindestens ein Heteroatom enthält, unterbrochen ist und/oder mit mindestens einem Halogenatom substituiert ist;
X bedeutet:
- eine geradkettige oder verzweigte Alkylgruppe mit 1 bis 14 Kohlenstoffatomen oder eine Alkenylgruppe mit 2 bis 14 Kohlenstoffatomen, die gegebenenfalls durch mindestens ein Heteroatom und/oder eine Gruppe, die mindestens ein Heteroatom enthält, unterbrochen und/oder mit mindestens einem Heteroatom und/oder einer Gruppe, die mindestens ein Heteroatom enthält, substituiert und/oder mit mindestens einem Halogenatom substituiert ist;
- eine 5- oder 6-gliedrige heterocyclische Gruppe, die gegebenenfalls mit mindestens einer geradkettigen oder verzweigten Alkylgruppe mit 1 bis 14 Kohlenstoffatomen, die gegebenenfalls mit mindestens einem Heteroatom substituiert ist; mit mindestens einer geradkettigen oder verzweigten Aminoalkylgruppe mit 1 bis 4 Kohlenstoffatomen, die gegebenenfalls mit mindestens einem Heteroatom substituiert ist; mit mindestens einem Halogenatom substituiert ist;
- eine aromatische Gruppe oder eine kondensierte oder nicht kondensierte, diaromatische Gruppe, die gegebenenfalls durch eine Alkylgruppe mit 1 bis 4 Kohlenstoffatomen separiert ist, wobei die Arylgruppe(n) gegebenenfalls mit mindestens einem Halogenatom oder mit mindestens einer Alkylgruppe mit 1 bis 10 Kohlenstoffatomen substituiert ist, die gegebenenfalls mit mindestens einem Heteroatom und/oder einer Gruppe, die mindestens ein Heteroatom enthält, substituiert und/ oder durch mindestens ein Heteroatom und/oder eine Gruppe, die mindestens ein Heteroatom enthält, unterbrochen ist;
- eine Dicarbonylgruppe;
- wobei die Gruppe X eine oder mehrere kationische Ladungen umfassen kann;
wobei a 0 oder 1 beträgt;
die Gruppen Y⁻, die gleich oder verschieden sind, ein organisches oder anorganisches Anion;
wobei n eine ganze Zahl von mindestens 2 und höchstens der Anzahl der in der fluoreszierenden Verbindung vorliegenden kationischen Ladungen ist; 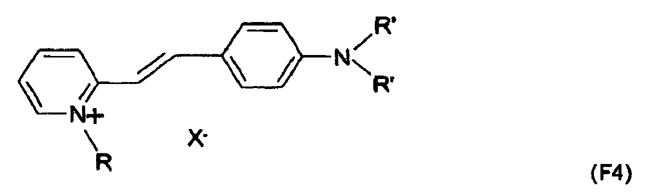 wobei in der Formel R eine Methylgruppe oder eine Ethylgruppe bedeutet; R' eine Methylgruppe ist, X⁻ ein Anion vom Typ Chlorid, Iodid, Sulfat, Methosulfat, Acetat oder Perchlorat bedeutet.

24. Verfahren nach einem der Ansprüche 20 bis 23, **dadurch gekennzeichnet, dass** die Haare pigmentierte oder künstlich gefärbte Keratinfasern sind:

25. Verfahren nach einem der Ansprüche 20 bis 24, **dadurch gekennzeichnet, dass** die Haare einen Farbton von 4 oder darunter aufweisen.

26. Verfahren zum Färben mit aufhellender Wirkung nach einem der Ansprüche 20 bis 25, **dadurch gekennzeichnet, dass** die folgenden Schritte durchgeführt werden:
a) auf die Fasern wird eine Zusammensetzung nach einem der Ansprüche 20 bis 23 während einer Zeitspanne aufgebracht, die ausreichend ist, um die gewünschte Färbung und Aufhellung zu erzielen,
b) die Fasern werden gegebenenfalls gespült,
c) die Fasern werden gegebenenfalls mit Haarwaschmittel gewaschen und gespült,
d) die Fasern werden getrocknet oder trocknen gelassen.

27. Verfahren nach Anspruch 26, **dadurch gekennzeichnet, dass** es einen vorbereiteten Schritt umfasst, der darin besteht, einerseits eine Zusammensetzung nach einem der Ansprüche 20 bis 23 und andererseits eine Zusammensetzung, die in einem kosmetisch akzeptablen Medium mindestens ein Oxidationsmittel enthält, getrennt voneinander aufzubewahren und sie bei der Anwendung, bevor das Gemisch während einer Zeitspanne, die ausreichend ist, um die gewünschte Färbung zu erzielen, auf die Fasern aufgebracht wird, zu vermischen, worauf gespült, gegebenenfalls mit Haarwaschmittel gewaschen, nochmals gespült und getrocknet wird.

28. Verfahren zum Färben von dunkler Haut mit aufhellender Wirkung, **dadurch gekennzeichnet, dass** auf die Haut eine Zusammensetzung nach einem der Ansprüche 1 bis 12 aufgetragen und die Haut anschließend getrocknet oder trocknen gelassen wird.

29. Vorrichtung mit mehreren Abteilungen zum Färben und Bleichen von Keratinfasern, die mindestens eine Abteilung aufweist, die eine Zusammensetzung nach einem der Ansprüche 1 bis 12 und 14 bis 17 enthält, und mindestens eine weitere Abteilung besitzt, die eine Zusammensetzung enthält, die mindestens ein Oxidationsmittel umfasst.
